# EUROPÄISCHE PATENTANMELDUNG

(11) **EP 2 030 967 A1**
(43) Veröffentlichungstag der Anmeldung: **04.03.2009**
(21) Anmeldenummer: 07115440.5
(22) Anmeldetag: 31.08.2007
(51) Int. Cl.: C07C 251/08, C08G 18/28, C08G 18/48, C09J 175/02

(54) **Hydroxylgruppen aufweisende Aldimine und Aldimin enthaltende Zusammensetzungen**

(71) Anmelder: Sika Technology AG, 6340 Baar (CH)
(72) Erfinder: Burckhardt, Urs, 8049 Zürich (CH)
(74) Vertreter: Sika Patent Attorneys

(57) **Zusammenfassung**

Die vorliegende Erfindung betrifft Aldimine der Formel (I), welche mindestens eine Hydroxylgruppe aufweisen, sowie härtbare Zusammensetzungen enthaltend diese Aldimine. Die Aldimine lassen sich auf einfache Art und Weise herstellen und sind breit einsetzbar. Die bei der Hydrolyse entstehenden Aldehyde lassen sich über die Hydroxylgruppen in ein Polymer einbauen und weisen tertiäre Aminogruppen auf, welche katalytisch wirken können.

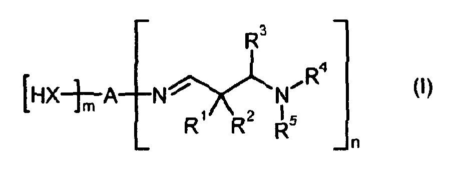

## Beschreibung

### Technisches Gebiet

Die Erfindung betrifft das Gebiet der Aldimine.

### Stand der Technik

Aldimine sind Kondensationsprodukte aus primären Aminen und Aldehyden und stellen eine seit langem bekannte Stoffklasse dar. Bei Kontakt mit Wasser können Aldimine zu den entsprechenden Aminen und Aldehyden hydrolysieren. Aufgrund dieser Eigenart können sie als geschützte Form von Aminen, beziehungsweise von Aldehyden, verwendet werden. So werden Aldimine beispielsweise in der Polyurethanchemie eingesetzt, wo sie als durch Feuchtigkeit aktivierbare Vernetzer, sogenannte "blockierte Amine", als Härter für ein- oder zweikomponentige Isocyanatgruppen aufweisende Zusammensetzungen dienen.

Die Verwendung von Aldiminen als Härter in Isocyanatgruppen aufweisenden Zusammensetzungen weist einige Vorteile auf. Zum einen weisen die Aldimine gegenüber den Isocyanatgruppen eine moderate, gut beherrschbare Reaktivität auf, während die entsprechenden freien Amine viel zu schnell reagieren und als Härter im allgemeinen nicht einsetzbar sind. Zum anderen verhindert die Anwesenheit der Aldimine die direkte, Kohlendioxid (CO₂) erzeugende Reaktion der Isocyanatgruppen mit Feuchtigkeit und unterdrückt damit die Entstehung von unerwünschten Gasblasen in der Zusammensetzung weitgehend.

Der Einsatz von Aldiminen als Härter in Isocyanatgruppen aufweisenden Zusammensetzungen kann aber auch Probleme verursachen, insbesondere aufgrund der Tatsache, dass bei der Aushärtung solcher Zusammensetzungen Aldehyde freigesetzt werden, welche nicht in das entstehende Polymer eingebaut werden. In Abhängigkeit der verwendeten Aldehyde können die Zusammensetzungen einen sehr starken Geruch aufweisen, welcher für viele Anwendungen nicht tolerierbar ist. Überdies können die Aldehyde durch Migrationseffekte aus der Zusammensetzung ausschwitzen oder deren mechanische Festigkeit oder Beständigkeit vermindern.

WO 2004/013088 A1 beschreibt geruchsfreie Polyaldimine, welche aus primären Polyaminen und geruchsfreien Aldehyden hergestellt werden. WO 2007/036571 A1 beschreibt geruchsfreie Aldimine enthaltend mindestens eine Hydroxyl-, Mercapto- oder sekundäre Aminogruppe, welche ebenfalls ausgehend von geruchsfreien Aldehyden erhältlich sind. Die aus diesen Aldiminen freigesetzten Aldehyde verbleiben aufgrund ihrer geringen Flüchtigkeit weitgehend in der ausgehärteten Zusammensetzung und können dort eine weichmachende und/oder die Festigkeit vermindernde Wirkung haben. Das relativ hohe Molekulargewicht dieser Aldehyde führt überdies dazu, dass die Aldimine in relativ grosser Menge eingesetzt werden müssen, was ihren Einsatz teuer machen kann.

### Darstellung der Erfindung

Aufgabe der vorliegenden Erfindung ist es, neue Aldimine zur Verfügung zu stellen, welche sich als Härter in härtbaren Zusammensetzungen, insbesondere in Isocyanatgruppen aufweisenden Zusammensetzungen mit vorteilhaften Eigenschaften einsetzen lassen, wobei die freigesetzten Aldehyde bei der Aushärtung der Zusammensetzungen in das entstehende Polymer eingebaut werden.

Überraschenderweise wurde gefunden, dass Aldimine nach Anspruch 1 diese Aufgabe lösen. Es handelt sich dabei um thermisch stabile, bei Raumtemperatur meist flüssige Verbindungen, welche kaum Geruch aufweisen und aus gut erhältlichen Grundstoffen in einem einfachen Verfahren herstellbar sind. Sie weisen tertiäre Aminogruppen von relativ niedriger Basizität auf und können in chemischen Reaktionssystemen katalytisch wirken. Weiterhin tragen sie Hydroxylgruppen, welche für weiterführende Reaktionen, beispielsweise mit Isocyanatgruppen, zur Verfügung stehen.

Diese Aldimine sind beispielsweise geeignet als Härter für härtbare Zusammensetzungen, welche gegenüber Aminen reaktive Gruppen, wie Epoxidgruppen, Anhydridgruppen und insbesondere Isocyanatgruppen, enthalten. In Isocyanatgruppen aufweisenden Zusammensetzungen werden die bei der Aushärtung aus den Aldiminen freigesetzten Aldehyde über deren Hydroxylgruppen kovalent in das entstehende Polyurethanpolymer eingebaut und verbleiben so vollständig in der Zusammensetzung.

Ein weiterer Gegenstand der Erfindung sind Aldimine gemäss Anspruch 15, welche Umsetzungsprodukte der Aldimine gemäss Anspruch 1 darstellen.

Ein weiterer Gegenstand der Erfindung sind härtbare Zusammensetzungen enthaltend die beschriebenen Aldimine nach Anspruch 17.

Schliesslich bilden ein Verfahren zur Herstellung der Aldimine gemäss Anspruch 12, Verwendungen gemäss Anspruch 16, sowie ein Artikel gemäss Anspruch 29 weitere Gegenstände der vorliegenden Erfindung.

Weitere Aspekte der Erfindung sind Gegenstand weiterer unabhängiger Ansprüche. Besonders bevorzugte Ausführungsformen der Erfindung sind Gegenstand der abhängigen Ansprüche.

### Wege zur Ausführung der Erfindung

Gegenstand der Erfindung sind Aldimine der Formel (I), 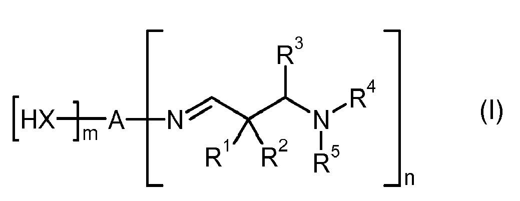 wobei
A entweder für den Rest eines Amins nach Entfernung von n primären aliphatischen Aminogruppen und m HX-Gruppen steht,
oder zusammen mit R⁷ für einen (n+2)-wertigen Kohlenwasserstoffrest mit 3 bis 20 C-Atomen, welcher gegebenenfalls mindestens ein Heteroatom, insbesondere in der Form von Ether-Sauerstoff oder tertiärem Amin-Stickstoff, enthält, steht;
n für 1 oder 2 oder 3 oder 4 steht;
m für 0 oder 1 oder 2 oder 3 oder 4 steht;
R¹ und R² entweder
   unabhängig voneinander jeweils für einen einwertigen Kohlenwasserstoffrest mit 1 bis 12 C-Atomen stehen,
   oder zusammen für einen zweiwertigen Kohlenwasserstoffrest mit 4 bis 12 C-Atomen, der Teil eines, gegebenenfalls substituierten, carbocyclischen Rings mit 5 bis 8, bevorzugt 6, C-Atomen ist, stehen;
R³ für ein Wasserstoffatom oder eine Alkylgruppe oder eine Arylalkylgruppe oder eine Alkoxycarbonylgruppe steht;
R⁴ und R⁵ entweder
   unabhängig voneinander jeweils für eine Methylgruppe oder einen einwertigen aliphatischen, cycloaliphatischen oder arylaliphatischen Rest mit 2 bis 12 C-Atomen stehen, welcher gegebenenfalls Hydroxylgruppen aufweist, und welcher gegebenenfalls Heteroatome in der Form von Ether-Sauerstoff oder tertiärem Amin-Stickstoff enthält,
mit der Massgabe, dass R⁴ mindestens eine Hydroxylgruppe aufweist, oder
   zusammen für einen mindestens eine Hydroxylgruppe aufweisenden zweiwertigen aliphatischen Rest mit 4 bis 12 C-Atomen, der Teil eines, gegebenenfalls substituierten, heterocyclischen Rings mit 5 bis 8, bevorzugt 6, Ringatomen ist, wobei dieser Ring gegebenenfalls weitere Heteroatome in der Form von Ether-Sauerstoff oder tertiärem Amin-Stickstoff enthält, stehen;
X für O oder S oder N-R⁶ oder N-R⁷ steht,
   wobei R⁶
   entweder für einen einwertigen Kohlenwasserstoffrest mit 1 bis 20 C-Atomen, welcher gegebenenfalls mindestens eine Carbonsäureester-, Nitril-, Nitro-, Phosphonsäureester-, Sulfon- oder Sulfonsäureester-Gruppe aufweist, steht,
   oder für einen Substituenten der Formel (II) steht, 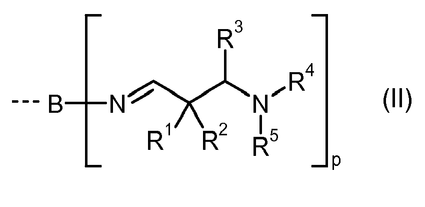 wobei
   p für 0 oder für eine ganze Zahl von 1 bis 10'000 steht, und
   B für einen (p+1)-wertigen Kohlenwasserstoffrest, welcher gegebenenfalls Ether-Sauerstoff, tertiären Amin-Stickstoff, Hydroxylgruppen, sekundäre Aminogruppen oder Mercaptogruppen enthält, steht; und
R⁷ zusammen mit A für einen (n+2)-wertigen Kohlenwasserstoffrest mit 3 bis 20 C-Atomen, welcher gegebenenfalls mindestens ein Heteroatom, insbesondere in Form von Ether-Sauerstoff oder tertiärem Amin-Stickstoff, enthält, steht.

Die gestrichelten Linien in den Formeln in diesem Dokument stellen jeweils die Bindung zwischen einem Substituenten und dem zugehörigen Molekülrest dar.

Der Begriff "primäre Aminogruppe" bezeichnet im vorliegenden Dokument eine Aminogruppe in der Form einer NH₂-Gruppe, die an einen organischen Rest gebunden ist. Der Begriff "sekundäre Aminogruppe" bezeichnet eine Aminogruppe, in der das Stickstoffatom an zwei organische Reste gebunden ist, welche auch gemeinsam Teil eines Rings sein können. Der Begriff "tertiäre Aminogruppe" bezeichnet eine Aminogruppe, in der das Stickstoffatom an drei organische Reste gebunden ist, wobei zwei dieser Reste auch gemeinsam Teil eines Rings sein können (=tertiärer Amin-Stickstoff).

Als "aliphatisch" wird ein Amin oder eine Aminogruppe bezeichnet, worin das Stickstoffatom ausschliesslich an aliphatische, cycloaliphatische oder arylaliphatische Reste gebunden ist.

Der Begriff "aktiver Wasserstoff" bezeichnet im vorliegenden Dokument das Wasserstoffatom einer Hydroxyl-, einer Mercapto- oder einer sekundären Aminogruppe.

Bevorzugt stehen R¹ und R² jeweils für eine Methylgruppe.

Bevorzugt steht R³ für ein Wasserstoffatom.

Bevorzugt stehen R⁴ und R⁵ jeweils für eine 2-Hydroxyethylgruppe oder je für eine 2-Hydroxypropylgruppe.

Bevorzugte Aldimine der Formel (I) sind solche, bei welchen die Reste R⁴ und R⁵ zusammen mindestens zwei Hydroxylgruppen aufweisen und der Rest A mindestens difunktionell ist. Solche bevorzugten Aldimine der Formel (I) stellen Aldimine der Formel (I') dar, 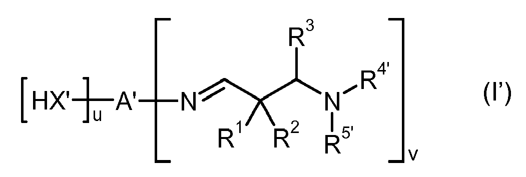 wobei
A' entweder für den Rest eines Amins nach Entfernung von v primären aliphatischen Aminogruppen und u HX'-Gruppen steht,
oder zusammen mit R^{7'} für einen (v+2)-wertigen Kohlenwasserstoffrest mit 3 bis 20 C-Atomen, welcher gegebenenfalls mindestens ein Heteroatom, insbesondere in der Form von Ether-Sauerstoff oder tertiärem Amin-Stickstoff, enthält, steht;
u für 1 oder 2 oder 3 oder 4 steht, und
v für 0 oder 1 oder 2 oder 3 oder 4 steht,
mit der Massgabe, dass u+v für 2 oder 3 oder 4 oder 5 steht;
R^{4'} und R^{5'} entweder
   unabhängig voneinander jeweils für eine Methylgruppe oder einen einwertigen aliphatischen, cycloaliphatischen oder arylaliphatischen Rest mit 2 bis 12 C-Atomen stehen, welcher gegebenenfalls Hydroxylgruppen aufweist, und welcher gegebenenfalls Heteroatome in der Form von Ether-Sauerstoff oder tertiärem Amin-Stickstoff enthält,
mit der Massgabe, dass R^{4'} mindestens eine Hydroxylgruppe aufweist und dass R^{4'} und R^{5'} zusammen mindestens zwei Hydroxylgruppen aufweisen, oder
   zusammen für einen mindestens zwei Hydroxylgruppen aufweisenden zweiwertigen aliphatischen Rest mit 4 bis 12 C-Atomen, der Teil eines, gegebenenfalls substituierten, heterocyclischen Rings mit 5 bis 8, bevorzugt 6, Ringatomen ist, wobei dieser Ring gegebenenfalls weitere Heteroatome in der Form von Ether-Sauerstoff oder tertiärem Amin-Stickstoff enthält, stehen;
X' für O oder S oder N-R^{6'} oder N-R^{7'} steht,
   wobei R^{6'}
   entweder für einen einwertigen Kohlenwasserstoffrest mit 1 bis 20 C-Atomen, welcher gegebenenfalls mindestens eine Carbonsäureester-, Nitril-, Nitro-, Phosphonsäureester-, Sulfon- oder Sulfonsäureester-Gruppe aufweist, steht,
   oder für einen Substituenten der Formel (II') steht, 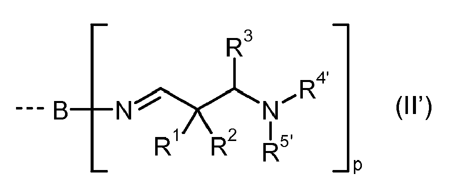 und
   R^{7'} zusammen mit A' für einen (v+2)-wertigen Kohlenwasserstoffrest mit 3 bis 20 C-Atomen, welcher gegebenenfalls mindestens ein Heteroatom, insbesondere in Form von Ether-Sauerstoff oder tertiärem Amin-Stickstoff, enthält, steht; und
B, p, R¹, R² und R³ die bereits erwähnten Bedeutungen aufweisen.

Bevorzugt steht (u+v) in Formel (I') für 2 oder 3.

Bevorzugt weisen R^{4'} und R^{5'} in Formel (I') zusammen zwei Hydroxylgruppen auf. Solche bevorzugten Aldimine der Formel (I') enthalten in einer Ausführungsform einen Rest R^{4'} mit zwei Hydroxylgruppen und einen Rest R^{5'} ohne Hydroxylgruppe; oder in einer weiteren Ausführungsform einen Rest R^{4'} mit einer Hydroxylgruppen und einen Rest R^{5'} mit einer Hydroxylgruppe.

Besonders bevorzugte Aldimine der Formel (I') stellen in einer Ausführungsform Aldimine der Formel (I a) dar, 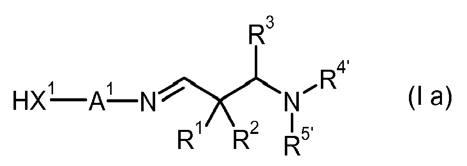 wobei
A¹ keinen aktiven Wasserstoff und keine primären Aminogruppen aufweist und entweder
für einen zweiwertigen Kohlenwasserstoffrest mit 2 bis 20 C-Atomen, welcher gegebenenfalls mindestens ein Heteroatom, insbesondere in der Form von Ether-Sauerstoff oder tertiärem Amin-Stickstoff, enthält, steht, oder
zusammen mit R⁹ für einen dreiwertigen Kohlenwasserstoffrest mit 3 bis 20 C-Atomen, welcher gegebenenfalls mindestens ein Heteroatom, insbesondere in der Form von Ether-Sauerstoff oder tertiärem Amin-Stickstoff, enthält, steht;
X¹ für O oder S oder N-R⁸ oder N-R⁹ steht,
   wobei R⁸
   entweder für einen einwertigen Kohlenwasserstoffrest mit 1 bis 20 C-Atomen, welcher gegebenenfalls mindestens eine Carbonsäureester-, Nitril-, Nitro-, Phosphonsäureester-, Sulfon- oder Sulfonsäureester-Gruppe aufweist, steht,
   oder für einen Substituenten der Formel (II a) steht, 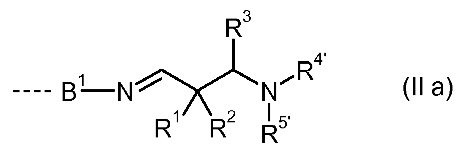 wobei B¹ für einen zweiwertigen, gegebenenfalls Ether-Sauerstoff oder tertiären Amin-Stickstoff aufweisenden Kohlenwasserstoffrest mit 2 bis 12 C-Atomen steht; und
   R⁹ zusammen mit A¹ für einen dreiwertigen Kohlenwasserstoffrest mit 3 bis 20 C-Atomen, welcher gegebenenfalls mindestens ein Heteroatom, insbesondere in der Form von Ether-Sauerstoff oder tertiärem Amin-Stickstoff, enthält, steht;
und R¹, R², R³, R^{4'} und R^{5'} die bereits genannten Bedeutungen aufweisen.

Besonders bevorzugte Aldimine der Formel (I') stellen in einer weiteren Ausführungsform Aldimine der Formel (I b) dar, 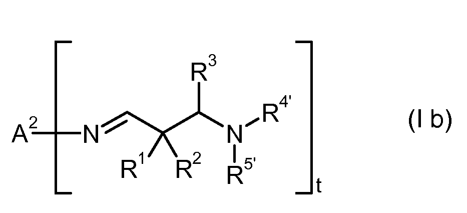 wobei
t für 2 oder 3 steht;
A² für den Rest eines Polyamins mit t primären Aminogruppen nach Entfernung von t primären Aminogruppen steht und keinen aktiven Wasserstoff enthält; und R¹, R², R³, R^{4'} und R^{5'} die bereits genannten Bedeutungen aufweisen.

Aldimine der Formel (I) sind erhältlich aus der Umsetzung von mindestens einem Amin **B** der Formel (III) mit mindestens einem sterisch gehinderten, aliphatischen, mindestens eine Hydroxylgruppe aufweisenden Aldehyd **ALD** der Formel (IV), 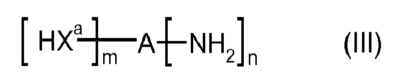 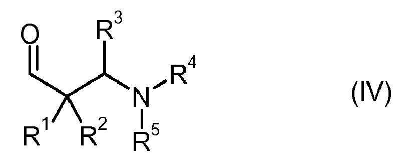 wobei
X^{a} für O oder S oder N-R^{6a} oder N-R⁷steht,
   wobei R^{6a} entweder für einen einwertigen Kohlenwasserstoffrest mit 1 bis 20 C-Atomen, welcher gegebenenfalls mindestens eine Carbonsäureester-, Nitril-, Nitro-, Phosphonsäureester-, Sulfon- oder Sulfonsäureester-Gruppe aufweist, steht,
   oder für einen Substituenten der Formel (III') steht; 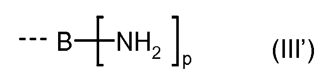
und m, n, p, A, B, R¹, R², R³, R⁴ und R⁵ die bereits genannten Bedeutungen aufweisen.

Die Umsetzung zwischen einem Amin **B** der Formel (III) und einem Aldehyd **ALD** der Formel (IV) erfolgt in einer Kondensationsreaktion unter Abspaltung von Wasser. Solche Kondensationsreaktionen sind bestens bekannt und beschrieben, beispielsweise in Houben-Weyl, "Methoden der organischen Chemie", Vol. XI/2, Seite 73ff. Der Aldehyd **ALD** wird hierbei in Bezug auf die primären Aminogruppen des Amins **B** stöchiometrisch oder in stöchiometrischem Überschuss eingesetzt. Üblicherweise werden solche Kondensationsreaktionen in Anwesenheit eines Lösemittels durchgeführt, mittels welchem das bei der Reaktion entstehende Wasser azeotrop entfernt wird. Zur Herstellung der Aldimine der Formel (I) wird jedoch ein Herstellverfahren ohne Verwendung von Lösemitteln bevorzugt, wobei das bei der Kondensation gebildete Wasser direkt mittels Anlegen von Vakuum aus der Reaktionsmischung entfernt wird. Durch die lösemittelfreie Herstellung erübrigt sich ein Abdestillieren des Lösemittels nach erfolgter Herstellung, was den Herstellungsprozess vereinfacht. Zudem ist das Aldimin so frei von Lösemittelrückständen, welche einen störenden Geruch verursachen könnten.

Als Amin **B** geeignet sind in einer Ausführungsform primäre Amine, wie beispielsweise die Isomeren Butyl-,Pentyl-,Hexyl-,Heptyl-,Octyl-, Nonyl-, Decyl-, Undecyl-, Dodecyl- und Tridecylamine, Alkoxyalkylamine wie 2-Methoxyethylamin,2-Ethoxyethylamin, 3-Methoxypropylamin, 3-Ethoxypropylamin, 3(2-Ethyl-hexyloxy)propylamin und höhere Homologe wie zum Beispiel 3-(2-Methoxy-ethoxy)propylamin, Cyclohexylamin, Benzylamin und 2-Phenylethylamin.

Als Amin **B** weiterhin geeignet sind Verbindungen, welche neben einer oder mehreren primären Aminogruppen mindestens eine einen aktiven Wasserstoff tragende Reaktivgruppe in der Form einer Hydroxyl-, Mercapto-oder sekundäre Aminogruppe aufweisen. Beispiele für Amine **B** mit mehr als einer aktiven Wasserstoff tragenden Reaktivgruppe sind

- mehr als eine sekundäre Aminogruppe und eine oder mehrere primäre Aminogruppen tragende aliphatische Amine, wie N,N'-Bis-(3-amino-propyl)ethylendiamin, Triethylentetramin (TETA), Tetraethylenpentamin (TEPA), Pentaethylenhexamin und höhere Homologe linearer Polyethylenamine, N,N'-Bis-(3-aminopropyl)-ethylendiamin, Produkte aus der mehrfachen Cyanoethylierung oder Cyanobutylierung und anschliessender Hydrierung von primären Di- und Polyaminen mit mehreren primären Aminogruppen, wie N,N'-Bis-(3-aminopropyl)-ethylendiamin, N,N'-Bis-(3-aminopropyl)-1,4-diaminobutan, N,N'-Bis-(3-aminopropyl)-2-methyl-1,5-pentandiamin, N,N'-Bis-(3-amino-1-ethylpropyl)-2-methyl-1,5-pentandiamin sowie Polyethylenimine unterschiedlichen Polymerisationsgrades (Molmassen-Bereich 500 bis 1'000'000 g/mol), wie sie beispielsweise unter dem Handelsnamen Lupasol^{®} von BASF in reiner Form oder als wässrige Lösungen erhältlich sind, wobei diese Polyethylenimine neben primären und sekundären auch tertiäre Aminogruppen enthalten;
- mehr als eine Hydroxylgruppe und eine oder mehrere primäre Aminogruppen tragende Hydroxyamine, insbesondere Derivate von polyalkoxylierten drei- oder höherwertigen Alkoholen oder von polyalkoxylierten Polyaminen, sowie Aminozucker, beispielsweise Glucosamin oder Galactosamin;
- mindestens eine Hydroxyl- und mindestens eine sekundäre Aminogruppen tragende Hydroxypolyamine aus der Cyanoethylierung oder Cyanobutylierung und anschliessender Hydrierung von Hydroxyaminen wie N-Hydroxyethyl-1,2-ethandiamin, N-Hydroxypropyl-1,2-ethandiamin, N-Hydroxyethyl-1,3-propandiamin, N3-Hydroxyethyl-1,3-pentandiamin.

Als Amin **B** geeignet sind weiterhin Polyamine, welche zwei oder mehr primäre aliphatische Aminogruppen aufweisen. Beispiele für Amine **B** mit mehr als drei primären aliphatischen Aminogruppen sind Polyvinylamine oder primäre Aminogruppen tragende Copolymerisate, beispielsweise aus Allylamin und (Meth)acrylaten.

Als Amin **B** besonders geeignet sind zum einen Amine **B1** der Formel (III a), 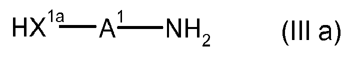 wobei
X^{1a} für O oder S oder N-R^{8a} oder N-R⁹ steht,
   wobei R^{8a} entweder für einen einwertigen Kohlenwasserstoffrest mit 1 bis 20 C-Atomen, welcher gegebenenfalls mindestens eine Carbonsäureester-, Nitril-, Nitro-, Phosphonsäureester-, Sulfon- oder Sulfonsäureester-Gruppe aufweist, steht,
   oder für einen Substituenten der Formel (III a') steht; 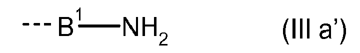 und A¹, B¹ und R⁹ die bereits genannten Bedeutungen aufweisen.

Die Amine **B1** sind insbesondere geeignet zur Herstellung von Aldiminen der Formel (I a).

Beispiele für Amine **B1** sind
- Verbindungen mit einer oder zwei primären aliphatischen und einer sekundären Aminogruppe, wie beispielsweise N-Methyl-1,2-ethandiamin, N-Ethyl-1,2-ethandiamin, N-Butyl-1,2-ethandiamin, N-Hexyl-1,2-ethandiamin, N-(2-Ethylhexyl)-1,2-ethandiamin, N-Cyclohexyl-1,2-ethandiamin, 4-Aminomethyl-piperidin, 3-(4-Aminobutyl)-piperidin, N-(2-Aminoethyl)piperazin, Diethylentriamin (DETA), Bis-hexamethylentriamin (BHMT), 3-(2-Aminoethyl)amino-propylamin; Di- und Triamine aus der Cyanoethylierung oder Cyanobutylierung und anschliessender Hydrierung von primären Mono- und Diaminen, beispielsweise N-Methyl-1,3-propandiamin, N-Ethyl-1,3-propandiamin, N-Butyl-1,3-propandiamin, N-Hexyl-1,3-propandiamin, N-(2-Ethylhexyl)-1,3-propandiamin, N-Dodecyl-1,3-propandiamin, N-Cyclohexyl-1,3-propandiamin, 3-Methyl-amino-1-pentylamin, 3-Ethylamino-1-pentylamin, 3-Butylamino-1-pentylamin, 3-Hexylamino-1-pentylamin, 3-(2-Ethylhexyl)amino-1-pentylamin, 3-Dodecyl-amino-1-pentylamin, 3-Cyclohexylamino-1-pentylamin, Dipropylentriamin (DPTA), N3-(3-Aminopentyl)-1,3-pentandiamin, N5-(3-Aminopropyl)-2-methyl-1,5-pentandiamin, N5-(3-Amino-1-ethylpropyl)-2-methyl-1,5-pentandiamin, und Fettdiamine wie N-Cocoalkyl-1,3-propandiamin, N-Oleyl-1,3-propandiamin, N-Soyaalkyl-1,3-propandiamin, N-Talgalkyl-1,3-propandiamin oder N-(C₁₆₋₂₂-Alkyl)-1,3-propandiamin, wie sie beispielsweise unter dem Handelsnamen Duomeen^{®} von Akzo Nobel erhältlich sind; die Produkte aus der Michael-artigen Addition von aliphatischen primären Di- oder Triaminen mit Acrylnitril, Malein- oder Fumarsäurediestern, Citraconsäurediestern, Acryl- und Methacrylsäureestern, Acryl- und Methacrylsäureamiden und Itaconsäurediestern, umgesetzt im Molverhältnis 1:1;
- aliphatische Hydroxyamine, wie beispielsweise 2-Aminoethanol, 2-Methylaminoethanol, 1-Amino-2-propanol, 3-Amino-1-propanol, 4-Amino-1-butanol, 4-Amino-2-butanol, 2-Amino-2-methylpropanol, 5-Amino-1-pentanol, 6-Amino-1-hexanol, 7-Amino-1-heptanol, 8-Amino-1-octanol, 10-Amino-1-decanol, 12-Amino-1-dodecanol, 4-(2-Aminoethyl)-2-hydroxyethylbenzol, 3-Aminomethyl-3,5,5-trimethyl-cyclohexanol; eine primäre Aminogruppe tragende Derivate von Glykolen wie Diethylenglykol, Dipropylenglykol, Dibutylenglykol und höheren Oligomeren und Polymeren dieser Glykole, beispielsweise 2-(2-Aminoethoxy)-ethanol, Triethylenglykol-monoamin, α-(2-Hydroxymethylethyl)-ω-(2-aminomethylethoxy)-poly(oxy(methyl-1,2-ethandiyl)); eine Hydroxylgruppe und eine primäre Aminogruppen tragende Derivate von polyalkoxylierten drei- oder höherwertigen Alkoholen; Produkte aus der einfachen Cyanoethylierung und anschliessender Hydrierung von Glykolen, beispielsweise 3-(2-Hydroxyethoxy)-propylamin, 3-(2-(2-Hydroxyethoxy)-ethoxy)-propylamin und 3-(6-Hydroxyhexyloxy)-propylamin;
- aliphatische Mercaptoamine, wie beispielsweise 2-Aminoethanthiol (Cysteamin), 3-Aminopropanthiol, 4-Amino-1-butanthiol, 6-Amino-1-hexanthiol, 8-Amino-1-octanthiol, 10-Amino-1-decanthiol, 12-Amino-1-dodecanthiol und Aminothiozucker wie 2-Amino-2-deoxy-6-thioglucose.

Als Amin **B1** bevorzugt sind N-Methyl-1,2-ethandiamin, N-Ethyl-1,2-ethandiamin, N-Cyclohexyl-1,2-ethandiamin, N-Methyl-1,3-propandiamin, N-Ethyl-1,3-propandiamin, N-Butyl-1,3-propandiamin, N-Cyclohexyl-1,3-propandiamin, 4-Aminomethyl-piperidin, 3-(4-Aminobutyl)-piperidin, DETA, DPTA, BHMT und Fettdiamine wie N-Cocoalkyl-1,3-propandiamin, N-Oleyl-1,3-propandiamin, N-Soyaalkyl-1,3-propandiamin und N-Talgalkyl-1,3-propandiamin; Produkte aus der Michael-artigen Additionsreaktion von aliphatischen primären Diaminen mit Malein- und Fumarsäurediestern, Acryl- und Methacrylsäureestern, Acryl- und Methacrylsäureamiden, bevorzugt mit Maleinsäurediestern, insbesondere Maleinsäuredimethyl-, -diethyl-, -dipropyl- und -dibutylester, und mit Acrylsäureestern, insbesondere Acrylsäuremethylester, umgesetzt im Molverhältnis 1:1; sowie aliphatische Hydroxy- oder Mercaptoamine, in denen die primäre Aminogruppe von der Hydroxyl- oder Mercaptogruppe durch eine Kette von mindestens 5 Atomen, oder durch einen Ring, getrennt sind, insbesondere 5-Amino-1-pentanol, 6-Amino-1-hexanol und höhere Homologe davon, 4-(2-Aminoethyl)-2-hydroxyethylbenzol, 3-Aminomethyl-3,5,5-trimethyl-cyclohexanol, 2-(2-Aminoethoxy)-ethanol, Triethylenglykol-monoamin und höhere Oligo- und Polymere davon, 3-(2-Hydroxyethoxy)-propylamin, 3-(2-(2-Hydroxyethoxy)-ethoxy)-propylamin und 3-(6-Hydroxyhexyloxy)-propylamin.

Als Amin **B1** besonders bevorzugt sind Amine, welche ausgewählt sind aus der Gruppe bestehend aus N-Methyl-1,2-ethandiamin, N-Ethyl-1,2-ethan-diamin, N-Cyclohexyl-1,2-ethandiamin, N-Methyl-1,3-propandiamin, N-Ethyl-1,3-propandiamin, N-Butyl-1,3-propandiamin, N-Cyclohexyl-1,3-propandiamin, 4-Aminomethyl-piperidin, 3-(4-Aminobutyl)-piperidin, DETA, DPTA, BHMT, Fettdiamine wie N-Cocoalkyl-1,3-propandiamin, N-Oleyl-1,3-propandiamin, N-Soyaalkyl-1,3-propandiamin und N-Talgalkyl-1,3-propandiamin, 5-Amino-1-pentanol, 6-Amino-1-hexanol, 4-(2-Aminoethyl)-2-hydroxyethylbenzol, 3-Aminomethyl-3,5,5-trimethyl-cyclohexanol, 2-(2-Aminoethoxy)-ethanol, Triethylenglykol-monoamin, 3-(2-Hydroxyethoxy)-propylamin, 3-(2-(2-Hydroxyethoxy)-ethoxy)-propylamin und 3-(6-Hydroxyhexyloxy)-propylamin.

Als Amin **B** besonders geeignet sind zum anderen Amine **B2** der Formel (III b), 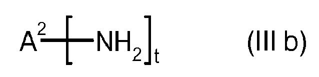 wobei A² und t die bereits genannten Bedeutungen aufweisen.

Die Amine **B2** sind insbesondere geeignet zur Herstellung von Aldiminen der Formel (I b).

Beispiele für Amine **B2** sind
- aliphatische, cycloaliphatische oder arylaliphatische Diamine, beispielsweise Ethylendiamin, 1,2-Propandiamin, 1,3-Propandiamin, 2-Methyl-1,2-propandiamin, 2,2-Dimethyl-1,3-propandiamin, 1,3-Butandiamin, 1,4-Butandiamin, 1,3-Pentandiamin (DAMP), 1,5-Pentandiamin, 1,5-Diamino-2-methylpentan (MPMD), 1,6-Hexandiamin, 2,5-Dimethyl-1,6-hexandiamin, 2,2,4- und 2,4,4-Trimethylhexamethylendiamin (TMD), 1,7-Heptandiamin, 1,8-Octandiamin, 1,9-Nonandiamin, 1,10-Decandiamin, 1,11-Undecandiamin, 1,12-Dodecandiamin und Methyl-bis-(3-aminopropyl)amin, 1,2-, 1,3- und 1,4-Diaminocyclohexan, Bis-(4-aminocyclohexyl)-methan, Bis-(4-amino-3-methylcyclohexyl)-methan, Bis-(4-amino-3-ethylcyclohexyl)-methan, Bis-(4-amino-3,5-dimethylcyclohexyl)-methan, Bis-(4-amino-3-ethyl-5-methylcyclohexyl)-methan (M-MECA), 1-Amino-3-aminomethyl-3,5,5-trimethylcyclohexan (= Isophorondiamin oder IPDA), 2- und 4-Methyl-1,3-diaminocyclohexan und Mischungen davon, 1,3- und 1,4-Bis-(aminomethyl)cyclohexan, 2,5(2,6)-Bis-(aminomethyl)-bicyclo[2.2.1]heptan (NBDA), 3(4),8(9)-Bis-(aminomethyl)-tricyclo[5.2.1.0^{2,6}]decan, 1,4-Diamino-2,2,6-trimethylcyclohexan (TMCDA), 3,9-Bis-(3-aminopropyl)-2,4,8,10-tetraoxaspiro[5.5]undecan sowie 1,3- und 1,4-Xylylendiamin;
- Ethergruppen-haltige aliphatische Diamine, beispielsweise Bis-(2-aminoethyl)ether, 3,6-Dioxaoctan-1,8-diamin, 4,7-Dioxadecan-1,10-diamin, 4,7-Dioxadecan-2,9-diamin, 4,9-Dioxadodecan-1,12-diamin, 5,8-Dioxadode-can-3,10-diamin und höhere Oligomere dieser Diamine, Bis-(3-aminopropyl)-polytetrahydrofurane und andere Polytetrahydrofuran-diamine mit Molekulargewichten im Bereich von beispielsweise 350 bis 5200, sowie Polyoxyalkylen-Diamine. Letztere stellen typischerweise Produkte aus der Aminierung von Polyoxyalkylen-Diolen dar und sind beispielsweise erhältlich unter dem Namen Jeffamine^{®} (von Huntsman Chemicals), unter dem Namen Polyetheramin (von BASF) oder unter dem Namen PC Amine^{®} (von Nitroil). Insbesondere geeignete Polyoxyalkylen-Diamine sind Jeffamine^{®} D-230, Jeffamine^{®} D-400, Jeffamine^{®} D-2000, Jeffamine^{®} D-4000, Jeffamine^{®} XTJ-511, Jeffamine^{®} ED-600, Jeffamine^{®} ED-900, Jeffamine^{®} ED-2003, Jeffamine^{®} XTJ-568, Jeffamine^{®} XTJ-569, Jeffamine^{®} XTJ-523, Jeffamine^{®} XTJ-536, Jeffamine^{®} XTJ-542, Jeffamine^{®} XTJ-559; Polyetheramin D 230, Polyetheramin D 400 und Polyetheramin D 2000, PC Amine^{®} DA 250, PC Amine^{®} DA 400, PC Amine^{®} DA 650 und PC Amine^{®} DA 2000;
- aliphatische Triamine wie 4-Aminomethyl-1,8-octandiamin, 1,3,5-Tris(aminomethyl)-benzol, 1,3,5-Tris-(aminomethyl)-cyclohexan;
- Polyoxyalkylen-Triamine, welche typischerweise Produkte aus der Aminierung von Polyoxyalkylen-Triolen darstellen und beispielsweise erhältlich sind unter dem Handelsnamen Jeffamine^{®} (von Huntsman Chemicals), unter dem Namen Polyetheramin (von BASF) oder unter dem Namen PC Amine^{®} (von Nitroil), wie zum Beispiel Jeffamine^{®} T-403, Jeffamine^{®} T-5000; Polyetheramin T403, Polyetheramin T5000; und PC Amine^{®} TA 403, PC Amine^{®} TA 5000.

Als Amin **B2** bevorzugt sind Polyamine, welche ausgewählt sind aus der Gruppe bestehend aus 1,6-Hexamethylendiamin, MPMD, DAMP, IPDA, TMD, 1,3-Xylylendiamin, 1,3-Bis-(aminomethyl)cyclohexan, Bis-(4-aminocyclohexyl)-methan, Bis-(4-amino-3-methylcyclohexyl)-methan, 3(4),8(9)-Bis-(aminomethyl)-tricyclo[5.2.1.0^{2,6}]decan, 1,2-, 1,3- und 1,4-Diaminocyclohexan, 1,4-Diamino-2,2,6-trimethylcyclohexan, 3,6-Dioxaoctan-1,8-diamin, 4,7-Dioxadecan-1,10-diamin, 4-Aminomethyl-1,8-octandiamin und Polyoxyalkylen-Polyamine mit zwei oder drei Aminogruppen, insbesondere die unter dem Handelsnamen Jeffamine^{®} erhältlichen Typen D-230, D-400, D-2000, T-403 und T-5000 von Huntsman und dazu analoge Verbindungen von BASF oder Nitroil.

Im Aldehyd **ALD** der Formel (IV) stehen R¹ und R² bevorzugt jeweils für eine Methylgruppe. R³ steht bevorzugt für ein Wasserstoffatom.

Bevorzugte Aldehyde **ALD** stellen mindestens zwei Hydroxylgruppen aufweisende Aldehyde **ALD1** der Formel (IV') dar, 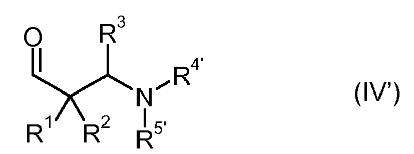 wobei R¹, R², R³, R^{4'} und R^{5'} die bereits genannten Bedeutungen aufweisen.

Bevorzugt weisen R^{4'} und R^{5'} in Formel (IV') zusammen zwei Hydroxylgruppen auf.

Aldehyde **ALD** der Formel (IV) sind insbesondere erhältlich als Produkt einer Mannich-Reaktion oder einer der Mannich-Reaktion analogen α-Aminoalkylierung, wie sie aus der Fachliteratur bekannt ist; sie können deshalb auch als Mannich-Basen bezeichnet werden. Ein Aldehyd **Y1** der Formel (V), ein Aldehyd **Y2** der Formel (VI) und ein sekundäres, aliphatisches, mindestens eine Hydroxylgruppe aufweisendes Amin **C** der Formel (VII) werden dabei unter Wasserabspaltung zum Aldehyd **ALD** der Formel (IV) umgesetzt, 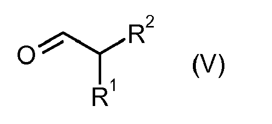 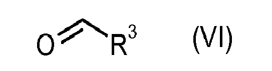 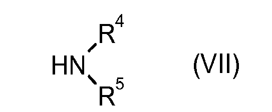 wobei R¹, R², R³, R⁴ und R⁵ die bereits genannten Bedeutungen aufweisen.

Diese Umsetzung kann entweder mit den freien Reagenzien **Y1, Y2** und **C** gemäss den Formeln (V), (VI) und (VII) geführt werden, oder die Reagenzien können teilweise oder vollständig in derivatisierter Form eingesetzt werden. So kann der Aldehyd **Y1** beispielsweise als Enolat, als Enolether, insbesondere als Silylenolether, oder als Enamin eingesetzt werden. Der Aldehyd **Y2** kann beispielsweise in Form eines Oligomeren - im Fall von Formaldehyd insbesondere als 1,3,5-Trioxan oder als Paraformaldehyd - oder als Hydrat, Hemiacetal, Acetal, N,O-Acetal, Aminal oder Hemiaminal eingesetzt werden. Das sekundäre, aliphatische, mindestens eine Hydroxylgruppe aufweisende Amin **C** schliesslich kann beispielsweise als Salz, insbesondere als Amin-Hydrochlorid oder als Amin-Hydrosulfat, eingesetzt werden. Es ist möglich, einen Teil der Reagenzien in freier Form und einen Teil in derivatisierter Form einzusetzen, oder nur von derivatisierten Formen auszugehen. Bei der Verwendung von Reagenzien in derivatisierter Form fällt der Aldehyd **ALD** unter Umständen ebenfalls in derivatisierter Form, beispielsweise als Salz, an; in diesem Fall kann er durch geeignete Aufarbeitung in die freie Form gemäss Formel (IV) übergeführt werden. Je nachdem kann es sinnvoll sein, in solchen Umsetzungsreaktionen zusätzlich Hilfsstoffe wie Lewissäuren oder Katalysatoren einzusetzen.

Weiterhin kann die Umsetzung als Eintopfreaktion geführt werden, in der alle drei Reagenzien gleichzeitig miteinander reagieren können; oder aber es kann ein stufenweises Vorgehen gewählt werden, indem zwei der Reagenzien vorgängig miteinander umgesetzt werden und das so erhaltene Zwischenprodukt anschliessend mit dem dritten Reagens umgesetzt wird, wobei das Zwischenprodukt isoliert werden kann oder nicht. Als solche Zwischenprodukte geeignet sind insbesondere Iminiumsalze, welche aus der Umsetzung eines Aldehyds **Y2,** in freier oder derivatisierter Form, mit einem Salz eines sekundären, aliphatischen, mindestens eine Hydroxylgruppe aufweisenden Amins **C** erhalten werden und welche sich mit einem Aldehyd **Y1,** in freier oder derivatisierter Form, zum entsprechenden Salz eines Aldehyds **ALD** der Formel (IV) umsetzen lassen. Ein solcherart stufenweises Vorgehen kann den Vorteil haben, mildere Reaktionsbedingungen zu ermöglichen und damit eine höhere Produktausbeute zu liefern.

Weiterhin kann die Umsetzung unter Verwendung von Lösemitteln, insbesondere polaren Lösemitteln wie Wasser oder Alkoholen, durchgeführt werden, oder die Umsetzung kann ohne Verwendung von Lösemitteln erfolgen.

In einer bevorzugten Ausführungsform wird die Umsetzung mit allen Reagenzien in freier Form als Eintopfreaktion geführt und der Aldehyd **ALD** nach erfolgter Umsetzung durch Destillation gereinigt. Bevorzugt werden dabei keine organischen Lösemittel eingesetzt.

Als Aldehyd **Y1** der Formel (V) geeignet sind beispielsweise die folgenden: Isobutyraldehyd, 2-Methylbutyraldehyd, 2-Ethylbutyraldehyd, 2-Methylvaleraldehyd, 2-Ethylcapronaldehyd, Cyclopentancarboxaldehyd, Cyclohexancarboxaldehyd, 1,2,3,6-Tetrahydrobenzaldehyd, 2-Methyl-3-phenylpropionaldehyd, 2-Phenylpropionaldehyd und Diphenylacetaldehyd. Bevorzugt ist Isobutyraldehyd.

Als Aldehyd **Y2** der Formel (VI) geeignet sind beispielsweise die folgenden: Formaldehyd, Acetaldehyd, Propionaldehyd, Butyraldehyd, Isobutyraldehyd, Phenylacetaldehyd, Benzaldehyd und substituierte Benzaldehyde sowie Glyoxylsäureester, insbesondere Glyoxylsäureethylester. Bevorzugt ist Formaldehyd.

Als Amin **C** der Formel (IV) geeignet sind sekundäre aliphatische Amine, welche eine Hydroxylgruppe aufweisen, wie beispielsweise diejenigen, welche ausgewählt sind aus der Gruppe bestehend aus Alkoxylaten von primären Aminen, wie 2-(N-Methylamino)ethanol, 2-(N-Ethylamino)ethanol, 2-(N-Propylamino)ethanol, 2-(N-Isopropylamino)ethanol, 2-(N-Butylamino)ethanol, 2-(N-Cyclohexylamino)ethanol, 3-(N-Methylamino)-2-propanol, 3-(N-Ethyl-amino)-2-propanol, 3-(N-Propylamino)-2-propanol, 3-(N-Isopropylamino)-2-propanol, 3-(N-Butylamino)-2-propanol, 3-(N-Cyclohexylamino)-2-propanol, 2-(N-Ethylaminoethoxy)ethanol; cycloaliphatische Hydroxyamine wie 2-Pyrrolidinmethanol, 3-Hydroxy-pyrrolidin, 2-Piperidin-methanol, 3- oder 4-Hydroxy-piperidin und 1-(2-Hydroxyethyl)-piperazin.

Als Amin **C** der Formel (IV) besonders geeignet sind sekundäre aliphatische Amine **C1,** welche mindestens zwei Hydroxylgruppen aufweisen. Die Amine **C1** sind insbesondere geeignet zur Herstellung der bevorzugten Aldehyde **ALD1** der Formel (IV').

Geeignete Amine **C1** mit zwei Hydroxylgruppen sind insbesondere ausgewählt aus der Gruppe bestehend aus Diethanolamin, Dipropanolamin, Diisopropanolamin, 3-(2-Hydroxyethylamino)-1-propanol und 3-(2-Hydroxy-propylamino)-1-propanol, N-Methyl-2,3-dihydroxypropylamin, 3,4-Dihydroxypyrrolidin, 2,5-Bis-(hydroxymethyl)-pyrrolidin, 2,6-Bis-(hydroxymethyl)-piperidin, 3,4- oder 3,5-Dihydroxy-piperidin, 2-(2,3-Dihydroxypropyl)-pyrrolidin und 2-(2,3-Dihydroxypropyl)-piperidin sowie die Umsetzungsprodukte aus Ammoniak mit zwei Molekülen, welche je eine Epoxidgruppe, insbesondere eine Glycidylether-Gruppe, aufweisen.

Geeignete Amine **C1** mit mehr als zwei Hydroxylgruppen sind beispielsweise die Folgenden: 2-(2,3-Dihydroxypropylamino)-ethanol, 3,4,5-Trihydroxy-piperidin, N,N-Bis-(2,3-dihydroxypropyl)-amin, 2,5-Bis-(2,3-dihydroxypropyl)-pyrrolidin und 2,6-Bis-(2,3-dihydroxypropyl)-piperidin.

Als Amin **C1** bevorzugt sind Diethanolamin und Diisopropanolamin.

Bevorzugte Aldehyde **ALD1** der Formel (IV') sind 3-(N-Bis(2-hydroxyethyl)amino)-2,2-dimethyl-propanal und 3-(N-Bis(2-hydroxy-2-methylethyl)-amino)-2,2-dimethyl-propanal.

Die Aldehyde **ALD** der Formel (IV) weisen eine Reihe von besonderen Eigenschaften auf. So besitzen sie eine gute thermische Stabilität, weil das C-Atom in α-Stellung zur Aldehydgruppe kein Wasserstoffatom trägt und deshalb die Eliminierung eines sekundären Amins unter Bildung eines Alkens nicht möglich ist. Ferner weisen sie eine überraschend gute Stabilität gegenüber Oxidation durch Luftsauerstoff auf. Weiterhin ist ihre Basizität überraschenderweise deutlich niedriger als für aliphatische Amine ähnlicher Struktur erwartet; der für die konjugierte Säure eines Aldehyds **ALD** gemessene *p*Kₐ-Wert liegt um ungefähr 2 Einheiten tiefer als jener der konjugierten Säure des zur Herstellung dieses Aldehyds **ALD** eingesetzten sekundären Amins **C.** Diese überraschenden Eigenschaften stehen eventuell in Zusammenhang mit einer intramolekularen 1,4-Wechselwirkung zwischen Amin- und Aldehydgruppe (Orbitalüberlappung zwischen dem freien Elektronenpaar des Stickstoffs und dem π- bzw. π*-Orbital des Carbonyls), wie sie von P.Y. Johnson et al. (J. Org. Chem., Vol. 40, Nr. 19, 1975; Seiten 2710-2720) anhand von NMR- und UV-spektroskopischen Untersuchungen an β-Aminoaldehyden postuliert wurde.

Schliesslich weisen die Aldehyde **ALD,** auch bei relativ niedrigem Molekulargewicht, keinen oder nur einen äusserst geringen Geruch auf. Diese für Aldehyde überraschende Eigenschaft der geringen Geruchsintensität kommt einerseits daher, dass die Aldehyde **ALD** aufgrund der vorhandenen OH-Gruppen relativ wenig flüchtig sind. Weiterhin wird der geringe Geruch vermutlich begünstigt durch die erwähnte intramolekulare 1,4-Wechselwirkung und durch die sterische Hinderung der Aldehydgruppe, welche an einem tertiären C-Atom steht.

Die Hydroxylgruppen der Aldehyde **ALD** ermöglichen Folgereaktionen zum Aufbau von weiter funktionalisierten Reaktionsprodukten. Die bevorzugten Aldehyde **ALD1** der Formel (IV'), welche mindestens zwei Hydroxylgruppen aufweisen, können insbesondere als Härter für Zusammensetzungen, welche gegenüber Hydroxylgruppen reaktive Komponenten, wie zum Beispiel Isocyanatgruppen, enthalten, eingesetzt werden.

Aldimine der Formel (I) lassen sich, wie bereits beschrieben, direkt aus Aminen **B** und Aldehyden **ALD** herstellen.

Aldimine der Formel (I), welche als Substituenten X einen Substituenten N-R⁶ aufweisen, lassen sich gegebenenfalls auf einem leicht anderen Weg als dem bisher beschriebenen herstellen. Dieser Syntheseweg besteht darin, dass ein Aldehyd **ALD** der Formel (IV) mit einem zwei- oder dreiwertigen, bevorzugt zweiwertigen, aliphatischen primären Amin, wie es vorgängig als Amin **B2** bereits beschrieben wurde, in einem ersten Schritt zu einem Zwischenprodukt umgesetzt wird, welches neben einer oder zwei Aldiminogruppen noch eine oder zwei, bevorzugt eine, primäre Aminogruppe enthält. Dieses Zwischenprodukt wird anschliessend in einem zweiten Schritt zu einem Aldimin der Formel (I) umgesetzt, indem die primäre Aminogruppe einfach alkyliert wird. Für die Alkylierung werden insbesondere Verbindungen mit nur einer aktivierten Doppelbindung eingesetzt, welche mit primären Aminen Michael-artige Additionsreaktionen eingehen können; solche Verbindungen werden im Folgenden als "Michael-Akzeptor" bezeichnet.

Die Umsetzung eines Aldehyds **ALD** mit einem Amin **B2** zum eine primäre Aminogruppe aufweisenden Zwischenprodukt erfolgt in einer Kondensationsreaktion unter Abspaltung von Wasser, wie sie weiter oben für die Umsetzung eines Aldehyds **ALD** mit einem Amin **B** der Formel (III) beschrieben wird. Die Stöchiometrie zwischen dem Aldehyd **ALD** und dem Amin **B2** wird dabei aber so gewählt, dass 1 mol Aldehyd **ALD** für 1 mol Amin **B2,** welches zwei primäre Aminogruppen enthält, eingesetzt wird, oder so, dass zwei mol Aldehyd **ALD** für ein mol Amin **B2,** welches drei primäre Aminogruppen enthält, eingesetzt wird. Das dabei eingesetzte Amin **B2** ist in Bezug auf die Aminogruppen bevorzugt asymmetrisch. Es wird ein lösemittelfreies Herstellverfahren bevorzugt, wobei das bei der Kondensation gebildete Wasser mittels Anlegen von Vakuum aus der Reaktionsmischung entfernt wird.

Die Umsetzung des eine primäre Aminogruppe aufweisenden Zwischenprodukts mit dem Michael-Akzeptor erfolgt beispielsweise dadurch, dass das Zwischenprodukt mit einer stöchiometrischen oder leicht überstöchiometrischen Menge des Michael-Akzeptors gemischt wird und die Mischung bei Temperaturen von 20 bis 110 °C bis zum vollständigen Umsatz des Zwischenprodukts zum Aldimin der Formel (I) erwärmt wird. Die Umsetzung erfolgt bevorzugt ohne die Verwendung von Lösemitteln.

Als Amin **B2** für diese Herstellung bevorzugt sind Diamine, in denen die primären Aminogruppen durch eine Kette von mindestens 5 Atomen, oder durch einen Ring, getrennt sind, insbesondere 1,5-Diamino-2-methylpentan, 1,6-Hexamethylendiamin, 2,2,4- und 2,4,4-Trimethylhexamethylendiamin und Mischungen davon, 1,10-Decandiamin, 1,12-Dodecandiamin, 1,3- und 1,4-Diaminocyclohexan, Bis-(4-aminocyclohexyl)-methan, Bis-(4-amino-3-methylcyclohexyl)-methan, 1-Amino-3-aminomethyl-3,5,5-trimethylcyclohexan, 1,3-und 1,4-Bis-(aminomethyl)cyclohexan, 2,5(2,6)-Bis-(aminomethyl)-bicyclo-[2.2.1]heptan, 3(4),8(9)-Bis-(aminomethyl)-tricyclo[5.2.1.0^{2,6}]decan, 1,4-Di-amino-2,2,6-trimethylcyclohexan (TMCDA), 3,9-Bis-(3-aminopropyl)-2,4,8,10-tetraoxaspiro[5.5]undecan, 1,3- und 1,4-Xylylendiamin, sowie die genannten Ethergruppen-haltigen aliphatischen Diamine und Polyoxyalkylen-Diamine.

Beispiele für geeignete Michael-Akzeptoren sind Malein- oder Fumarsäurediester wie Dimethylmaleinat, Diethylmaleinat, Dibutylmaleinat, Diethylfumarat; Citraconsäurediester wie Dimethylcitraconat; Acryl- oder Methacrylsäureester wie Methyl(meth)acrylat, Ethyl(meth)acrylat, Butyl(meth)acrylat, Lauryl(meth)acrylat, Stearyl(meth)acrylat, Tetrahydrofuryl(meth)acrylat, Isobor-nyl(meth)acrylat; Itaconsäurediester wie Dimethylitaconat; Zimtsäureester wie Methylcinnamat; Vinylphosphonsäurediester wie Vinylphosphonsäuredimethylester; Vinylsulfonsäureester, insbesondere Vinylsulfonsäurearylester; Vinylsulfone; Vinylnitrile wie Acrylnitril, 2-Pentennitril oder Fumaronitril; 1-Nitroethylene wie β-Nitrostyrol; und Knoevenagel-Kondensationsprodukte, wie beispielsweise solche aus Malonsäurediestern und Aldehyden wie Formaldehyd, Acetaldehyd oder Benzaldehyd. Bevorzugt sind Maleinsäurediester, Acrylsäureester, Phosphonsäurediester und Vinylnitrile.

Diejenigen Ausführungsformen von Aldiminen der Formel (I), welche mindestens eine Gruppe HX aufweisen, können gegebenenfalls im Gleichgewicht stehen mit cyclischen Formen, wie sie in Formel (VIII) beispielhaft für den Fall mit Index m=1 gezeigt sind. Diese cyclischen Formen sind im Fall von Aminoaldiminen cyclische Aminale, beispielsweise Imidazolidine oder Tetrahydropyrimidine; im Fall von Hydroxyaldiminen cyclische Aminoacetale, beispielsweise Oxazolidine oder Tetrahydrooxazine; im Fall von Mercaptoaldiminen cyclische Thioaminale, beispielsweise Thiazolidine oder Tetrahydrothiazine.

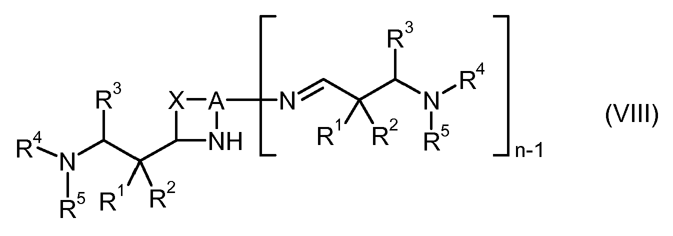

In der Formel (VIII) weisen n, A, X, R¹, R², R³, R⁴ und R⁵ die bereits genannten Bedeutungen auf.

Überraschenderweise neigen die meisten HX-Gruppen enthaltenden Aldimine der Formel (I) nicht zur Cyclisierung. Insbesondere für Aminoaldimine kann mittels IR- und NMR-spektroskopischen Methoden gezeigt werden, dass diese Verbindungen überwiegend in der offenkettigen, also der Aldimin-Form, vorliegen, während die cyclische, also die Aminal-Form, nicht oder nur in Spuren vorkommt. Auch Hydroxy- und Mercaptoamine, in denen die primäre Aminogruppe von der Hydroxyl- beziehungsweise der Mercaptogruppe durch eine Kette von mindestens 5 Atomen, oder durch einen Ring, getrennt sind, zeigen kaum Cyclisierung.

Bei den Aldiminen der Formel (I) handelt es sich um neue, bisher nicht beschriebene Verbindungen mit überraschenden Eigenschaften. Sie enthalten sterisch gehinderte Aldiminogruppen, welche am in α-Stellung stehenden C-Atom kein Wasserstoffatom aufweisen und deshalb nicht zu Enaminogruppen tautomerisieren können. Dadurch stellen diese Aldiminogruppen besonders gut geschützte ("blockierte") primäre Aminogruppen dar, welche in Gegenwart von Wasser mit gegenüber Aminen reaktiven Gruppen, wie Epoxidgruppen, Anydridgruppen und insbesondere Isocyanatgruppen, eine moderate, gut beherrschbare Reaktivität aufweisen, was in starkem Kontrast steht zur hohen Reaktivität der entsprechenden freien Aminogruppen, von welchen die Aldiminogruppen abgeleitet sind. Weiterhin weisen die Aldimine der Formel (I) eine tertiäre Aminogruppe auf, welche unter Umständen in chemischen Reaktionssystemen eine katalytische Wirkung entfalten kann; die von der tertiären Aminogruppe ausgehende Basizität der Aldimine der Formel (I) ist aber, wie bereits für die Aldehyde **ALD** der Formel (IV) erwähnt, eher niedrig. Weiterhin weisen die Aldimine der Formel (I), auch bei relativ niedrigem Molekulargewicht des zugrunde liegenden Aldehyds **ALD,** keinen oder nur einen geringen, aminartigen Geruch auf.

Die Aldimine der Formel (I) besitzen eine gute thermische Stabilität, da das C-Atom in α-Stellung zur Aldiminogruppe wie erwähnt kein Wasserstoffatom trägt und deshalb die Eliminierung eines sekundären Amins unter Bildung eines Alkens nicht möglich ist.

Die Aldimine der Formel (I) sind unter geeigneten Bedingungen lagerstabil. Bei Zutritt von Feuchtigkeit können ihre Aldiminogruppen über Zwischenstufen formal zu Aminogruppen hydrolysieren, wobei die entsprechenden, zur Herstellung der Aldimine verwendeten, Aldehyde ALD der Formel (IV) freigesetzt werden, welche, wie bereits beschrieben, geruchsarm oder geruchsfrei sind. Da diese Hydrolysereaktion reversibel ist und das chemische Gleichgewicht deutlich auf der Aldiminseite liegt, ist davon auszugehen, dass in Abwesenheit von gegenüber Aminen reaktiven Verbindungen nur ein Teil der Aldiminogruppen teilweise oder ganz hydrolysieren. Überraschenderweise lässt sich die Hydrolyse der Aldiminogruppen trotz der Anwesenheit von tertiären Aminogruppen mittels Säuren katalysieren.

Die Aldimine der Formel (I) weisen am Aldehyd-Teil mindestens eine Hydroxylgruppe auf. Dadurch sind weiterführende Reaktionen dieser Hydroxylgruppen mit gegenüber Hydroxylgruppen reaktiven Verbindungen möglich, insbesondere auch nach erfolgter Freisetzung des Aldehyds **ALD** bei der Hydrolyse der Aldiminogruppen.

Die Aldimine der Formel (I) sind aus gut erhältlichen AusgangsSubstanzen in einem relativ einfachen Verfahren herstellbar. Falls bei ihrer Herstellung dünnflüssige Amine **B** der Formel (III) verwendet wurden, sind die entsprechenden Aldimine der Formel (I) teilweise ebenfalls dünnflüssige Verbindungen.

Die Aldimine der Formel (I) lassen sich sehr breit verwenden. Sie lassen sich zum Beispiel überall dort einsetzen, wo sie als Quelle von Aldehyden **ALD** der Formel (IV) oder von Aminen **B** der Formel (III) dienen können. Insbesondere können sie in der Funktion von geschützten Aminen, beziehungsweise geschützten Aldehyden, in Aldehyd- und/oder Amin-reaktiven Systemen eingesetzt werden und dort bei Bedarf gezielt entschützt werden. Insbesondere finden sie in Systemen Anwendung, in welchen Verbindungen vorhanden sind, welche mit primären Aminen und/oder mit Hydroxylgruppen reagieren. Die Entschützung der primären Aminogruppen erfolgt hydrolytisch, beispielsweise durch Kontakt mit Wasser oder Feuchtigkeit, insbesondere Luftfeuchtigkeit. Überraschenderweise lässt sich die Hydrolyse der Aldiminogruppen trotz der Anwesenheit von tertiären Aminogruppen genauso mittels Säuren katalysieren wie bei Aldiminen ohne tertiäre Aminogruppen im Molekül.

Andererseits finden Aldimine der Formel (I) mit dem Index m grösser null Verwendung beim Aufbau von weiter funktionalisierten Reaktionsprodukten dieser Aldimine. So können Aldimine der Formel (I) mit dem Index m grösser null mit Verbindungen, welche mit der Gruppe HX reagieren können, umgesetzt werden, insbesondere wenn es sich bei den Gruppen HX um sekundäre Aminogruppen handelt. Zur Umsetzung mit der Gruppe HX geeignete Verbindungen tragen Reaktivgruppen wie zum Beispiel Isocyanatgruppen, Epoxidgruppen, Anhydridgruppen oder mehr oder weniger stark aktivierte Doppel- oder Dreifachbindungen wie (Meth)acrylatgruppen, Acrylamidgruppen, 1-Ethinylcarbonylgruppen, 1-Propinylcarbonylgruppen, Maleimidgruppen, Citraconimidgruppen, Vinylgruppen, Isopropenylgruppen oder Allylgruppen. Die Aldiminogruppen tragenden Umsetzungsprodukte aus solchen Additionsreaktionen lassen sich bei Bedarf zu Aldehyden **ALD** der Formel (IV) und Verbindungen mit primären Aminogruppen hydrolysieren und darauf für weitere Reaktionen nutzen, beispielsweise für Vernetzungsreaktionen, wobei sich die Hydrolysereaktion mittels Säuren katalysieren lässt.

Ferner können die Aldimine der Formel (I) als Katalysatoren in chemischen Reaktionssystemen wirken, beispielsweise in härtbaren, Isocyanatgruppen aufweisenden Zusammensetzungen, insbesondere um deren Aushärtungszeit zu verkürzen.

Schliesslich können die Aldimine der Formel (I) als Quelle für kationische Verbindungen verwendet werden, indem die tertiären Aminogruppen teilweise oder ganz zu Ammoniumgruppen protoniert oder zu quaternären Ammoniumgruppen alkyliert werden. Durch Protonierung oder Alkylierung von Aldiminen der Formel (I) sind Aldimine der Formel (IX) zugänglich. 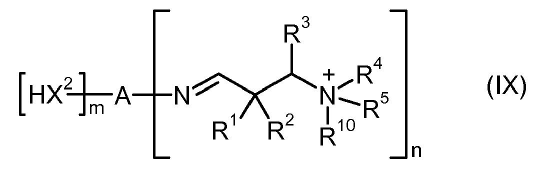 wobei
R¹⁰ für ein Wasserstoffatom oder für einen Alkyl-, Cycloalkyl- oder Arylalkylrest mit 1 bis 20 C-Atomen steht;
X² für O oder S oder N-R¹¹ oder N-R⁷ steht,
   wobei R¹¹
   entweder für einen einwertigen Kohlenwasserstoffrest mit 1 bis 20 C-Atomen, welcher gegebenenfalls mindestens eine Carbonsäureester-, Nitril-, Nitro-, Phosphonsäureester-, Sulfon- oder Sulfonsäureester-Gruppe aufweist, steht,
   oder für einen Substituenten der Formel (IX') steht; 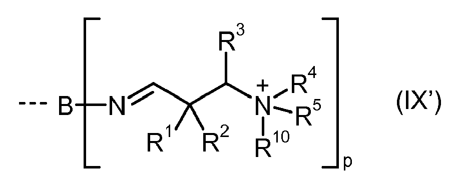
und m, n, p, A, B, R¹, R², R³, R⁴, R⁵ und R⁷ die bereits genannten Bedeutungen aufweisen.

Aldimine der Formel (IX) sind weiterhin erhältlich ausgehend von einem der vorgängig erwähnten Aminen **B** der Formel (III) und einem Aldehyd **ALD** der Formel (IV), wobei die tertiären Aminogruppen des Aldehyds **ALD** vor der Umsetzung mit dem Amin **B** teilweise oder ganz protoniert oder alkyliert werden.

Zur Protonierung der Aldimine der Formel (I) oder der Aldehyde **ALD** können beliebige Bronsted-Säuren eingesetzt werden, beispielsweise Salzsäure, Schwefelsäure, Phosphorsäure, Carbonsäuren wie Essigsäure oder Benzoesäure sowie Sulfonsäuren wie Methansulfonsäure oder p-Toluolsulfonsäure. Zur Alkylierung der Aldimine der Formel (I) oder der Aldehyde **ALD** können bekannte Alkylierungsmittel, insbesondere Methylierungsmittel, eingesetzt werden, beispielsweise Methyliodid, Dimethylsulfat, Dimethylphosphonat, Diazomethan, Fluorschwefelsäuremethylester oder Trimethyloxonium-tetrafluoroborat.

Es ist dem Fachmann klar, dass zu einem kationischen Aldimin der Formel (IX) auch ein Anion gehört, das die positive Ladung des Aldimins ausgleicht.

Besonders geeignet sind die Aldimine der Formel (I) oder der Formel (IX), insbesondere die bevorzugten, mindestens zwei Hydroxylgruppen aufweisenden Aldimine der Formel (I'), zur Verwendung als Bestandteil von Zusammensetzungen basierend auf Isocyanaten oder Epoxidharzen, insbesondere für Anwendungen wie Klebstoffe, Dichtstoffe, Vergussmassen, Beschichtungen, Bodenbeläge, Anstriche, Lacke, Primer und Schäume. Bevorzugt enthalten solche Zusammensetzungen mindestens eine Säure, insbesondere eine organische Carbon- oder Sulfonsäure, oder eine zu diesen Säuren hydrolysierbare Verbindung, wobei die Säure überraschenderweise die Hydrolyse der Aldiminogruppen trotz dem Vorhandensein von tertiären Aminogruppen katalysiert.

Insbesondere eignen sich die Aldimine der Formel (I) oder die Aldimine der Formel (IX), insbesondere die mindestens zwei Hydroxylgruppen aufweisenden Aldimine der Formel (I'), als Härter oder als Vorläufer für Härter für ein- oder zweikomponentige, Isocyanatgruppen aufweisende Zusammensetzungen wie Klebstoffe, Dichtstoffe, Vergussmassen, Beschichtungen, Bodenbeläge, Anstriche, Lacke, Primer und Schäume. Die bei der Hydrolyse der Aldiminogruppen freigesetzten Aldehyde **ALD** der Formel (IV) reagieren dabei über ihre Hydroxylgruppen mit den Isocyanatgruppen und werden somit kovalent in das bei der Aushärtung entstehende Polymer eingebaut. Im Fall der Verwendung von Aldiminen der Formel (I') werden Aldehyde **ALD1,** welche mindestens zwei Hydroxylgruppen tragen, freigesetzt; diese können ihrerseits ebenfalls als Härter dienen, indem sie zur Kettenverlängerung und/oder Vernetzung des entstehenden Polymers beitragen und nicht zu Kettenabbrüchen führen.

Wie bereits erwähnt, enthalten die Aldimine der Formel (I) sterisch gehinderte und nicht zu Enaminogruppen tautomerisierbare Aldiminogruppen, welche besonders gut geschützte ("blockierte") primäre Aminogruppen darstellen. Diese reagieren in Anwesenheit von Wasser mit vorhandenen Isocyanatgruppen, wobei ihre Reaktivität im Vergleich mit den entsprechenden freien primären Aminogruppen stark vermindert ist, so dass solche Systeme eine gut handhabbare Aushärtungsgeschwindigkeit aufweisen.

Die Hydroxylgruppen der freigesetzten Aldehyde **ALD** reagieren ebenfalls mit vorhandenen Isocyanatgruppen und werden somit wie erwähnt kovalent in das bei der Aushärtung entstehende Polymer eingebaut, was sehr vorteilhaft ist. Durch den Einbau der Aldehyde verursachen diese in der Zusammensetzung keine nachteiligen Effekte, wie beispielsweise erhöhten Schwund, Immissionen in die Umgebungsluft, insbesondere von unangenehmen Gerüchen, oder Migrationeffekte wie Ausschwitzungen; ebenso haben sie keine nachteiligen Einfluss auf die mechanischen Eigenschaften der Zusammensetzung, beispielsweise indem sie weichmachend wirken oder die Beständigkeit der Zusammensetzung gegenüber Umwelteinflüssen wie Hitze oder UV-Strahlung vermindern würden. Wie bereits erwähnt, sind Aldimine der Formel (I') bevorzugt, da die daraus freigesetzten Aldehyde **ALD1** mindestens zwei Hydroxylgruppen tragen und somit bei der Aushärtung von Isocyanatgruppen aufweisende Zusammensetzungen als mindestens difunktionelle Härter unter Kettenverlängerung oder Vernetzung kovalent in das entstehende Polymer eingebaut werden.

In Isocyanatgruppen aufweisenden Zusammensetzungen, welche als Härter Aldimine der Formel (I) oder der Formel (IX) enthalten, reagieren die Hydroxylgruppen und gegebenenfalls vorhandene sekundäre Aminogruppen direkt mit den Isocyanatgruppen, während die Aldiminogruppen in Anwesenheit von Wasser unter Hydrolyse mit den Isocyanatgruppen reagieren. Die Isocyanatgruppen reagieren dabei mit den durch die Hydrolyse der Aldiminogruppen formal frei werdenden primären Aminogruppen, wobei der entsprechende Aldehyd **ALD** in freier oder bereits adduktierter Form freigesetzt wird. Im Verhältnis zu den Aldiminogruppen, sekundären Aminogruppen und Hydroxylgruppen überschüssige Isocyanatgruppen reagieren direkt mit Feuchtigkeit unter Bildung von Harnstoffgruppen. Bei geeigneter Stöchiometrie zwischen Isocyanatgruppen und den Aldiminen der Formel (I) härtet die Zusammensetzung als Ergebnis dieser Reaktionen aus; dieser Prozess wird auch als Vernetzung bezeichnet. Die Reaktion der Isocyanatgruppen mit den hydrolysierenden Aldiminogruppen muss dabei nicht notwendigerweise über freie Aminogruppen erfolgen. Selbstverständlich sind auch Reaktionen mit Zwischenstufen der Hydrolysereaktion möglich. Beispielsweise ist es denkbar, dass eine hydrolysierende Aldiminogruppe in der Form eines Halbaminals direkt mit einer Isocyanatgruppe reagiert. Auch ist es für die Aushärtung einer solchen Zusammensetzung irrelevant, ob die Hydroxylgruppen im Aldehyd-Teil der Aldimine mit Isocyanatgruppen reagieren, bevor die Aldiminogruppen hydrolysieren, oder erst danach. Sobald genügend Feuchtigkeit in der Zusammensetzung vorhanden ist, beispielsweise aus der Luft in Form von Luftfeuchtigkeit, findet die Hydrolyse der Aldiminogruppen und Umsetzung der formal freigesetzten primären Aminogruppen mit Isocyanatgruppen statt, auch wenn die vorhandenen Hydroxylgruppen bereits mit Isocyanatgruppen umgesetzt sind. Überraschenderweise wird die säurekatalysierte Hydrolyse der Aldiminogruppen durch das Vorhandensein der tertiären Aminogruppen nicht beeinträchtigt.

Die tertiäre Aminogruppe der Aldimine der Formel (I) kann eine katalytische Wirkung auf die Reaktion der Isocyanatgruppen haben und kann deshalb die Vernetzung beschleunigen. Diese beschleunigende Wirkung wird zusätzlich unterstützt durch die Tatsache, dass die tertiären Aminogruppen im Aldehyd-Teil der Aldimine lokalisiert sind. Es ist dabei aber vorteilhaft, dass die Basizität der tertiären Aminogruppen vergleichsweise niedrig ist, da stark basische tertiäre Amine die direkte Reaktion der Isocyanatgruppen, insbesondere mit Wasser, übermässig beschleunigen können, was sich bei der Aushärtung störend auswirken kann. Bei der Hydrolyse der Aldiminogruppen werden Aldehyde **ALD** der Formel (IV) enthaltend die tertiäre Aminogruppe und mindestens eine Hydroxylgruppe freigesetzt. Die Aldehyde **ALD** werden über die Reaktion der Hydroxylgruppen mit Isocyanatgruppen kovalent in das entstehende Polymer eingebaut. Nach dem Einbau ins Polymer ist die katalytische Aktivität der tertiären Aminogruppe aufgrund deren eingeschränkten Beweglichkeit wesentlich reduziert, was für die Beständigkeit des Materials von Vorteil sein kann. Die bei der Hydrolyse der Aldiminogruppen entstehenden Aldehydgruppen bleiben bei der Aushärtung erhalten und können, falls gewünscht, für weiterführende Reaktionen verwendet werden.

Es ist auch möglich, die Aldimine der Formel (I) zusammen mit Wasser aufzubewahren. Erst wenn die Wasser-Aldimin-Mischung mit Isocyanatgruppen in Kontakt kommt, läuft die Hydrolyse vollständig ab. Die Reaktion zwischen Aldiminen der Formel (I) und Isocyanatgruppen ist nämlich auch dann stark verzögert im Vergleich zur Reaktion der entsprechenden freien Amine, wenn die Aldimine zusammen mit Wasser aufbewahrt wurden bzw. wenn Wasser im Überschuss vorhanden ist.

Ebenfalls möglich ist die Verwendung von Aldiminen der Formel (I) oder der Formel (IX) in Zusammensetzungen, welche unter dem Einfluss von Wärme aushärten, beispielsweise durch die Verwendung von Verbindungen mit thermisch labilen, blockierten Isocyanatgruppen. Weiterhin möglich ist die Verwendung von Aldiminen der Formel (I) oder der Formel (IX) in Zusammensetzungen, welche reaktive Warm- oder Heissschmelzklebstoffe darstellen. Solche Klebstoffe enthalten schmelzbare, insbesondere Isocyanatgruppen aufweisende, Verbindungen; sie sind bei Raumtemperatur fest und werden warm oder heiss appliziert.

Ein weiterer Gegenstand der Erfindung sind härtbare Zusammensetzungen, enthaltend mindestens ein Polyisocyanat und mindestens ein Aldimin der Formel (I) oder der Formel (IX).

Der Begriff "Polyisocyanat" umfasst im vorliegenden Dokument Verbindungen mit zwei oder mehr Isocyanatgruppen, unabhängig davon, ob es sich dabei um monomere Diisocyanate, oligomere Polyisocyanate oder Isocyanatgruppen aufweisende Polymere mit einem relativ hohen Molekulargewicht handelt.

Als Aldimin der Formel (I) geeignet sind die vorgängig detailliert beschriebenen Aldimine der Formel (I), respektive die bevorzugten Ausführungsformen davon, insbesondere die Aldimine der Formel (I'). Geeignete Aldimine der Formel (IX) wurden vorgängig bereits beschrieben.

Bevorzugt sind härtbare Zusammensetzungen enthaltend mindestens ein Polyisocyanat und mindestens ein Aldimin der Formel (I'), insbesondere mindestens ein Aldimin der Formel (I a) oder der Formel (I b).

In einer Ausführungsform ist die härtbare Zusammensetzung einkomponentig.

Als "einkomponentig" wird im vorliegenden Dokument eine härtbare Zusammensetzung bezeichnet, bei welcher alle Bestandteile der Zusammensetzung vermischt im gleichen Gebinde gelagert werden, und welche bei Raumtemperatur über einen längeren Zeitraum lagerstabil ist, sich also in ihren Anwendungs- oder Gebrauchseigenschaften durch die Lagerung nicht oder nur unwesentlich verändert, und welche nach der Applikation durch die Einwirkung von Feuchtigkeit und / oder Wärme aushärtet.

Die einkomponentige härtbare Zusammensetzung umfasst insbesondere mindestens ein Polyisocyanat, dessen Isocyanatgruppen insbesondere als blockierte Isocyanatgruppen vorliegen.

Unter einer "blockierten Isocyanatgruppe" wird im vorliegenden Dokument eine Isocyanatgruppe verstanden, welche durch die vorgängige Umsetzung einer freien Isocyanatgruppe mit einem aus dem Stand der Technik bekannten Blockierungsmittel, beispielsweise einem Phenol, einem Ketoxim, einem Pyrazol, einem Lactam oder einem Malonsäurediester, in ihrer Reaktivität gegenüber Nucleophilen so stark reduziert ist, dass sie zusammen mit geeigneten Härtern bei Raumtemperatur lagerstabil ist und erst unter der Einwirkung von Hitze und / oder Feuchtigkeit mit diesen Härtern zu reagieren beginnt, wobei das Blockierungsmittel je nach Typ freigesetzt oder nicht freigesetzt wird.

Geeignete Polyisocyanate mit blockierten Isocyanatgruppen sind kommerziell erhältlich, beispielsweise unter den Handelsnamen Desmocap^{®} 11, 12 und XP 2540 (alle von Bayer), Trixene^{®} BI 7641, BI 7642, BI 7770, BI 7771, BI 7772, BI 7774 und BI 7779 (alle von Baxenden), Vestanat^{®} B 1358A, B 1358/100 oder B 1370 (alle von Degussa), sowie Tolonate^{®} D2 (von Rhodia), oder können durch Umsetzung von Polyisocyanaten mit geeigneten Blockierungsmitteln nach Bedarf hergestellt werden.

Die einkomponentige härtbare Zusammensetzung kann feuchtigkeitshärtend und / oder hitzehärtend sein.

Unter einer "hitzehärtenden Zusammensetzung" wird im vorliegenden Dokument eine Zusammensetzung mit blockierten Isocyanatgruppen verstanden, bei welcher beim Erhitzen auf eine geeignete Temperatur, typischerweise im Bereich von 120 bis 200 °C, in speziellen Fällen schon bei Temperaturen ab 80 °C, die blockierten Isocyanatgruppen soweit aktiviert werden, dass mit geeigneten Härtern eine Vernetzung und somit Aushärtung eintritt. Dieser Vorgang wird auch als Einbrennen bezeichnet und erfolgt üblicherweise nach der Applikation der Zusammensetzung.

Typischerweise erfolgt die vollständige Aushärtung der beschriebenen einkomponentigen Zusammensetzung durch Einwirkung einer Kombination von Feuchtigkeit und Wärme.

In einer bevorzugten Ausführungsform ist die härtbare Zusammensetzung zweikomponentig.

Als "zweikomponentig" wird im vorliegenden Dokument eine härtbare Zusammensetzung bezeichnet, bei welcher die Bestandteile der Zusammensetzung in zwei verschiedenen Komponenten vorliegen, welche in voneinander getrennten Gebinden gelagert werden und welche jeweils für sich lagerstabil sind. Die beiden Komponenten werden als Komponente **K1** und als Komponente **K2** bezeichnet. Erst kurz vor oder während der Applikation der Zusammensetzung werden die beiden Komponenten miteinander vermischt, worauf die vermischte Zusammensetzung aushärtet, wobei die Aushärtung unter Umständen erst durch die Einwirkung von Feuchtigkeit und / oder Wärme abläuft oder vervollständigt wird.

Besonders bevorzugt sind zweikomponentige härtbare Zusammensetzungen bestehend aus einer Komponente **K1** und einer Komponente **K2,** wobei diese Zusammensetzungen mindestens ein Polyisocyanat **P** und mindestens ein Aldimin der Formel (I') enthalten. Bei deren Aushärtung werden mindestens zwei Hydroxylgruppen aufweisende Aldehyde **ALD1** der Formel (IV') freigesetzt, welche ihrerseits als Härter für das Polyisocyanat **P** wirken und unter Kettenverlängerung oder Vernetzung kovalent in das entstehende Polymer eingebaut werden.

Die Komponente **K1** der besonders bevorzugten härtbaren zweikomponentigen Zusammensetzung enthält mindestens ein Polyisocyanat **P.**

Als Polyisocyanat **P** geeignet ist in einer Ausführungsform ein Polyisocyanat **PI** in der Form eines monomeren Di- oder Triisocyanates oder eines Oligomeren eines monomeren Diisocyanates oder eines Derivates eines monomeren Diisocyanates.

Als monomere Di- oder Triisocyanate geeignet sind beispielsweise die folgenden: 1,4-Tetramethylendiisocyanat, 2-Methylpentamethylen-1,5-diisocyanat, 1,6-Hexamethylendiisocyanat (HDI), 2,2,4- und 2,4,4-Trimethyl-1,6-hexamethylendiisocyanat (TMDI), 1,10-Decamethylendiisocyanat, 1,12-Dodecamethylendiisocyanat, Lysin- und Lysinesterdiisocyanat, Cyclohexan-1,3- und - 1,4-diisocyanat, 1-Methyl-2,4- und -2,6-diisocyanatocyclohexan und beliebige Gemische dieser Isomeren (HTDI oder H₆TDI), 1-Isocyanato-3,3,5-trimethyl-5-isocyanatomethyl-cyclohexan (=Isophorondiisocyanat oder IPDI), Perhydro-2,4'- und -4,4'-diphenylmethandiisocyanat (HMDI oder H₁₂MDI), 1,4-Diiso-cyanato-2,2,6-trimethylcyclohexan (TMCDI), 1,3- und 1,4-Bis-(isocyanatomethyl)-cyclohexan, m- und p-Xylylendiisocyanat (m- und p-XDI), m- und p-Tetramethyl-1,3- und -1,4-xylylendiisocyanat (m- und p-TMXDI), Bis-(1-Isocya-nato-1-methylethyl)-naphthalin, Dimer- und Trimerfettsäureisocyanate wie 3,6-Bis-(9-isocyanatononyl)-4,5-di-(1-heptenyl)-cyclohexen (Dimeryldiisocyanat), α,α,α',α',α",α"-Hexamethyl-1,3,5-mesitylentriisocyanat, 2,4- und 2,6-Toluylendiisocyanat und beliebige Gemische dieser Isomeren (TDI), 4,4'-, 2,4'- und 2,2'-Diphenylmethandiisocyanat und beliebige Gemische dieser Isomeren (MDI), Gemische aus MDI und MDI-Homologen (polymeres MDI oder PMDI), 1,3- und 1,4-Phenylendiisocyanat, 2,3,5,6-Tetramethyl-1,4-diisocyanatobenzol, Naphthalin-1,5-diisocyanat (NDI), 3,3'-Dimethyl-4,4'-diisocyanatodiphenyl (TODI), Dianisidindiisocyanat (DADI), 1,3,5-Tris-(isocyanatomethyl)-benzol, Tris-(4-isocyanatophenyl)-methan und Tris-(4-isocyanatophenyl)-thiophosphat.

Als Polyisocyanat **PI** besonders geeignet sind Oligomere oder Derivate von monomeren Diisocyanaten, insbesondere von HDI, IPDI, TDI und MDI. Kommerziell erhältliche Typen sind insbesondere HDI-Biurete, beispielsweise als Desmodur^{®} N 100 und N 3200 (Bayer), Tolonate^{®} HDB und HDB-LV (Rhodia) und Duranate^{®} 24A-100 (Asahi Kasei); HDI-Isocyanurate, beispielsweise als Desmodur^{®} N 3300, N 3600 und N 3790 BA (alle von Bayer), Tolonate^{®} HDT, HDT-LV und HDT-LV2 (Rhodia), Duranate^{®} TPA-100 und THA-100 (Asahi Kasei) und Coronate^{®} HX (Nippon Polyurethane); HDI-Uretdione, beispielsweise als Desmodur^{®} N 3400 (Bayer); HDI-Iminooxadiazindione, beispielsweise als Desmodur^{®} XP 2410 (Bayer); HDI-Allophanate, beispielsweise als Desmodur^{®} VP LS 2102 (Bayer); IPDI-Isocyanurate, beispielsweise in Lösung als Desmodur^{®} Z 4470 (Bayer) oder in fester Form als Vestanat^{®} T1890/100 (Degussa); TDI-Oligomere, beispielsweise als Desmodur^{®} IL (Bayer); sowie gemischte Isocyanurate auf Basis TDI / HDI, zum Beispiel als Desmodur^{®} HL (Bayer). Weiterhin besonders geeeignet sind bei Raumtemperatur flüssige Formen von MDI (sogenanntes "modifiziertes MDI"), welche Gemische von MDI mit MDI-Derivaten, wie beispielsweise MDI-Carbodiimiden bzw. MDI-Uretoniminen oder MDI-Urethanen darstellen, bekannt beispielsweise unter Handelsnamen wie Desmodur^{®} CD, Desmodur^{®} PF, Desmodur^{®} PC (alle von Bayer), sowie Gemische aus MDI und MDI-Homologen (polymeres MDI oder PMDI), erhältlich unter Handelsnamen wie Desmodur^{®} VL, Desmodur^{®} VL50, Desmodur^{®} VL R10, Desmodur^{®} VL R20 und Desmodur^{®} VKS 20F (alle von Bayer), Isonate^{®} M 309, Voranate^{®} M 229 und Voranate^{®} M 580 (alle von Dow) oder Lupranat^{®} M 10 R (von BASF).

Die vorgenannten oligomeren Polyisocyanate **PI** stellen in der Praxis üblicherweise Gemische von Substanzen mit unterschiedlichen Oligomerisationsgraden und/oder chemischen Strukturen dar. Vorzugsweise weisen sie eine mittlere NCO-Funktionalität von 2.1 bis 4.0 auf und enthalten insbesondere Isocyanurat-, Iminooxadiazindion-, Uretdion-, Urethan-, Biuret-, Allophanat-, Carbodiimid-, Uretonimin- oder Oxadiazintrion-Gruppen. Bevorzugt weisen diese Oligomere einen niedrigen Gehalt an monomeren Diisocyanaten auf.

Als Polyisocyanat **PI** bevorzugt sind bei Raumtemperatur flüssige Formen von MDI, sowie die Oligomeren von HDI, IPDI und TDI, insbesondere die Isocyanurate.

Als Polyisocyanat **P** geeignet ist in einer weiteren Ausführungsform ein Isocyanatgruppen aufweisendes Polyurethanpolymer **PUP.**

Der Begriff "Polymer" umfasst im vorliegenden Dokument einerseits ein Kollektiv von chemisch einheitlichen, sich aber in Bezug auf Polymerisationsgrad, Molmasse und Kettenlänge unterscheidenden Makromolekülen, das durch eine Polyreaktion (Polymerisation, Polyaddition, Polykondensation) hergestellt wurde. Der Begriff umfasst andererseits auch Derivate eines solchen Kollektivs von Makromolekülen aus Polyreaktionen, Verbindungen also, die durch Umsetzungen, wie beispielsweise Additionen oder Substitutionen, von funktionellen Gruppen an vorgegebenen Makromolekülen erhalten wurden und die chemisch einheitlich oder chemisch uneinheitlich sein können. Der Begriff umfasst im Weiteren auch sogenannte Prepolymere, das heisst, reaktive oligomere Voraddukte, deren funktionelle Gruppen am Aufbau von Makromolekülen beteiligt sind.

Der Begriff "Polyurethanpolymer" umfasst sämtliche Polymere, welche nach dem sogenannten Diisocyanat-Polyadditions-Verfahren hergestellt werden. Dies schliesst auch solche Polymere ein, die nahezu oder gänzlich frei sind von Urethangruppen. Beispiele für Polyurethanpolymere sind Polyether-Polyurethane, Polyester-Polyurethane, Polyether-Polyharnstoffe, Polyharnstoffe, Polyester-Polyharnstoffe, Polyisocyanurate und Polycarbodiimide.

Ein geeignetes Isocyanatgruppen aufweisendes Polyurethanpolymer **PUP** ist erhältlich durch die Umsetzung von mindestens einem Polyol mit mindestens einem Polyisocyanat.

Als Polyole für die Herstellung eines Polyurethanpolymers **PUP** können beispielsweise die folgenden Polyole oder Mischungen davon eingesetzt werden:
- Polyetherpolyole, auch Polyoxyalkylenpolyole oder Oligoetherole genannt, welche Polymerisationsprodukte von Ethylenoxid, 1,2-Propylenoxid, 1,2-oder 2,3-Butylenoxid, Tetrahydrofuran oder Mischungen davon sind, eventuell polymerisiert mit Hilfe eines Startermoleküls wie beispielsweise Wasser, Ammoniak, 1,2-Ethandiol, 1,2- und 1,3-Propandiol, Neopentylglykol, Diethylenglykol, Triethylenglykol, die isomeren Dipropylenglykole und Tripropylenglykole, die isomeren Butandiole, Pentandiole, Hexandiole, Heptandiole, Octandiole, Nonandiole, Decandiole, Undecandiole, 1,3- und 1,4-Cyclohexandimethanol, Bisphenol A, hydriertes Bisphenol A, 1,1,1-Trimethylolethan, 1,1,1-Trimethylolpropan, Glycerin, Anilin, sowie Mischungen der vorgenannten Verbindungen. Eingesetzt werden können sowohl Polyoxyalkylenpolyole, die einen niedrigen Ungesättigtheitsgrad aufweisen (gemessen nach ASTM D-2849-69 und angegeben in Milliequivalent Ungesättigtheit pro Gramm Polyol (mEq/g)), hergestellt beispielsweise mit Hilfe von sogenannten Double Metal Cyanide Complex-Katalysatoren (DMC-Katalysatoren), als auch Polyoxyalkylenpolyole mit einem höheren Ungesättigtheitsgrad, hergestellt beispielsweise mit Hilfe von anionischen Katalysatoren wie NaOH, KOH, CsOH oder Alkalialkoholaten.

Als Polyetherpolyole besonders geeignet sind Polyoxyalkylendiole und -triole, insbesondere Polyoxyalkylendiole. Besonders geeignete Polyoxyalkylendi- und triole sind Polyoxyethylendi- und -triole sowie Polyoxypropylendi- und -triole.

Besonders geeignet sind Polyoxypropylendiole und -triole mit einem Ungesättigtheitsgrad tiefer als 0.02 mEq/g und einem Molekulargewicht im Bereich von 1000 bis 30'000 g/mol, sowie Polyoxypropylendiole und -triole mit einem Molekulargewicht von 400 bis 8'000 g/mol. Unter ,Molekulargewicht' oder ,Molgewicht' versteht man im vorliegenden Dokument stets das Molekulargewichtsmittel Mₙ. Insbesondere geeignet sind Polyoxypropylendiole mit einem Ungesättigtheitsgrad tiefer als 0.02 mEq/g und einem Molekulargewicht im Bereich von 1000 bis 12'000, insbesondere zwischen 1000 und 8000 g/mol. Solche Polyetherpolyole werden beispielsweise unter dem Handelsnamen Acclaim^{®} von Bayer vertrieben.

Ebenfalls besonders geeignet sind sogenannte "EO-endcapped" (ethylene oxide-endcapped) Polyoxypropylendiole und -triole. Letztere sind spezielle Polyoxypropylenpolyoxyethylenpolyole, die beispielsweise dadurch erhalten werden, dass reine Polyoxypropylenpolyole nach Abschluss der Polypropoxylierung mit Ethylenoxid alkoxyliert werden und dadurch primäre Hydroxylgruppen aufweisen.
- Styrol-Acrylnitril- oder Acrylnitril-Methylmethacrylat-gepfropfte Polyetherpolyole.
- Polyesterpolyole, auch Oligoesterole genannt, hergestellt nach bekannten Verfahren, insbesondere der Polykondensation von Hydroxycarbonsäuren oder der Polykondensation von aliphatischen und/oder aromatischen Polycarbonsäuren mit zwei- oder mehrwertigen Alkoholen.

Insbesondere geeignet sind Polyesterpolyole, welche hergestellt sind aus zwei- bis dreiwertigen, insbesondere zweiwertigen, Alkoholen, wie beispielsweise Ethylenglykol, Diethylenglykol, Propylenglykol, Dipropylenglykol, Neopentylglykol, 1,4-Butandiol, 1,5-Pentandiol, 3-Methyl-1,5-hexandiol, 1,6-Hexandiol, 1,8-Octandiol, 1,10-Decandiol, 1,12-Dodecandiol, 1,12-Hydroxystearylalkohol, 1,4-Cyclohexandimethanol, Dimerfettsäurediol (Dimerdiol), Hydroxypivalinsäureneopentylglykolester, Glycerin, 1,1,1-Trimethylolpropan oder Mischungen der vorgenannten Alkohole, mit organischen Di- oder Tricarbonsäuren, insbesondere Dicarbonsäuren, oder deren Anhydride oder Ester, wie beispielsweise Bernsteinsäure, Glutarsäure, Adipinsäure, Trimethyladipinsäure, Korksäure, Azelainsäure, Sebacinsäure, Dodecandicarbonsäure, Maleinsäure, Fumarsäure, Dimerfettsäure, Phthalsäure, Phthalsäureanhydrid, Isophthalsäure, Terephthalsäure, Dimethylterephthalat, Hexahydrophthalsäure, Trimellithsäure und Trimellithsäureanhydrid, oder Mischungen der vorgenannten Säuren, sowie Polyesterpolyole aus Lactonen wie beispielsweise aus ε-Caprolacton und Startern wie den vorgenannten zwei- oder dreiwertigen Alkoholen.

Besonders geeignete Polyesterpolyole sind Polyesterdiole.
- Polycarbonatpolyole, wie sie durch Umsetzung beispielsweise der oben genannten - zum Aufbau der Polyesterpolyole eingesetzten - Alkohole mit Dialkylcarbonaten, wie Dimethylcarbonat, Diarylcarbonaten, wie Diphenylcarbonat, oder Phosgen zugänglich sind.

Besonders geeignet sind Polycarbonatdiole.
- Als Polyole ebenfalls geeignet sind mindestens zwei Hydroxylgruppen tragende Blockcopolymere, welche mindestens zwei verschiedene Blöcke mit Polyether-, Polyester- und/oder Polycarbonatstruktur der oben beschriebenen Art aufweisen.
- Polyacrylat- und Polymethacrylatpolyole.
- Polyhydroxyfunktionelle Fette und Öle, beispielsweise natürliche Fette und Öle, inbesondere Ricinusöl; oder durch chemische Modifizierung von natürlichen Fetten und Ölen gewonnene - sogenannte oleochemische - Polyole, beispielsweise die durch Epoxidierung ungesättigter Öle und anschliessender Ringöffnung mit Carbonsäuren bzw. Alkoholen erhaltenen Epoxypolyester bzw. Epoxypolyether, oder durch Hydroformylierung und Hydrierung ungesättigter Öle erhaltene Polyole; oder aus natürlichen Fetten und Ölen durch Abbauprozesse wie Alkoholyse oder Ozonolyse und anschliessender chemischer Verknüpfung, beispielsweise durch Umesterung oder Dimerisierung, der so gewonnenen Abbauprodukte oder Derivaten davon erhaltene Polyole. Geeignete Abbauprodukte von natürlichen Fetten und Ölen sind insbesondere Fettsäuren und Fettalkohole sowie Fettsäureester, insbesondere die Methylester (FAME), welche beispielsweise durch Hydroformylierung und Hydrierung zu Hydroxyfettsäureestern derivatisiert werden können.
- Polykohlenwasserstoffpolyole, auch Oligohydrocarbonole genannt, wie beispielsweise polyhydroxyfunktionelle Polyolefine, Polyisobutylene, Polyisoprene; polyhydroxyfunktionelle Ethylen-Propylen-, Ethylen-Butylen-oder Ethylen-Propylen-Dien-Copolymere, wie sie beispielsweise von der Firma Kraton Polymers hergestellt werden; polyhydroxyfunktionelle Polymere von Dienen, insbesondere von 1,3-Butadien, welche insbesondere auch aus anionischer Polymerisation hergestellt sein können; polyhydroxyfunktionelle Copolymere aus Dienen wie 1,3-Butadien oder Diengemischen und Vinylmonomeren wie Styrol, Acrylnitril, Vinylchlorid, Vinylacetat, Vinylalkohol, Isobutylen und Isopren, beispielsweise polyhydroxyfunktionelle Acrylnitril/Butadien-Copolymere, welche zum Beispiel aus carboxylterminierten Acrylnitril/Butadien-Copolymeren (kommerziell erhältlich unter dem Namen Hycar^{®} CTBN von Noveon) und Epoxiden oder Aminoalkoholen herstellbar sind; sowie hydrierte polyhydroxyfunktionelle Polymere oder Copolymere von Dienen.

Diese genannten Polyole weisen bevorzugt ein mittleres Molekulargewicht von 250 - 30'000 g/mol, insbesondere von 400 - 20'000 g/mol, und weisen bevorzugt eine mittlere OH-Funktionalität im Bereich von 1.6 bis 3 auf.

Zusätzlich zu diesen genannten Polyolen können kleine Mengen von niedrigmolekularen zwei- oder mehrwertigen Alkoholen wie beispielsweise 1,2-Ethandiol, 1,2- und 1,3-Propandiol, Neopentylglykol, Diethylenglykol, Triethylenglykol, die isomeren Dipropylenglykole und Tripropylenglykole, die isomeren Butandiole, Pentandiole, Hexandiole, Heptandiole, Octandiole, Nonandiole, Decandiole, Undecandiole, 1,3- und 1,4-Cyclohexandimethanol, hydriertes Bisphenol A, dimere Fettalkohole wie beispielsweise Dimerfettsäurediole, 1,1,1-Trimethylolethan, 1,1,1-Trimethylolpropan, Glycerin, Pentaerythrit, niedrigmolekulare Alkoxylierungsprodukte der vorgenannten zwei- und mehrwertigen Alkohole, sowie Mischungen der vorgenannten Alkohole bei der Herstellung eines Polyurethanpolymers **PUP** mitverwendet werden.

Als Polyisocyanate für die Herstellung eines Polyurethanpolymers **PUP** können aliphatische, cycloaliphatische oder aromatische Polyisocyanate, insbesondere Diisocyanate, verwendet werden, beispielsweise die monomeren Diisocyanate, welche bereits als geeignete Polyisocyanate **PI** erwähnt wurden, sowie Oligomere und Polymere dieser monomeren Diisocyanate, sowie beliebige Mischungen dieser Isocyanate. Bevorzugt sind monomere Diisocyanate, insbesondere MDI, TDI, HDI und IPDI.

Die Herstellung eines Polyurethanpolymers **PUP** erfolgt in bekannter Art und Weise direkt aus den Polyisocyanaten und den Polyolen, oder durch schrittweise Adduktionsverfahren, wie sie auch als Kettenverlängerungsreaktionen bekannt sind.

In einer bevorzugten Ausführungsform wird das Polyurethanpolymer **PUP** über eine Reaktion von mindestens einem Polyisocyanat und mindestens einem Polyol hergestellt, wobei die Isocyanatgruppen gegenüber den Hydroxylgruppen im stöchiometrischen Überschuss vorliegen. Vorteilhaft beträgt das Verhältnis zwischen Isocyanat- und Hydroxylgruppen 1.3 bis 10, insbesondere 1.5 bis 5.

Vorteilhaft wird die Umsetzung bei einer Temperatur durchgeführt, bei der die verwendeten Polyole, Polyisocyanate und das gebildete Polyurethanpolymer flüssig vorliegen.

Das Polyurethanpolymer **PUP** weist ein Molekulargewicht von vorzugsweise über 500 g/mol, insbesondere ein solches zwischen 1000 und 30'000 g/mol, auf.

Weiterhin weist das Polyurethanpolymer **PUP** bevorzugt eine mittlere NCO-Funktionalität im Bereich von 1.8 bis 3 auf.

Als Polyisocyanat **P** geeignet sind schliesslich auch Gemische enthaltend ein Polyurethanpolymer **PUP** und ein Polyisocyanat **PI,** insbesondere einerseits Gemische enthaltend ein MDI-basiertes Polyurethanpolymer **PUP** und monomeres und/oder polymeres MDI, sowie andererseits Gemische enthaltend ein IPDI-basiertes Polyurethanpolymer **PUP** und monomeres und/oder oligomeres IPDI.

Die Komponente **K2** der besonders bevorzugten härtbaren zweikomponentigen Zusammensetzung enthält mindestens ein Aldimin der Formel (I'). Dafür geeignet sind die vorgängig beschriebenen Aldimine der Formel (I'), respektive die dafür bevorzugen Ausführungsformen, wie sie bereits im Detail beschrieben wurden, insbesondere Aldimine der Formel (I a) und Aldimine der Formel (I b).

Besonders bevorzugt sind Aldimine der Formel (I') mit Resten R^{4'} und R^{5'}, welche zusammen zwei Hydroxylgruppen aufweisen.

Gegebenenfalls enthält die Komponente **K2** weitere gegenüber Isocyanatgruppen reaktive Verbindungen, wie Polyamine, Polyole, Aminoalkohole, Polythiole, oder weitere blockierte Amine.

Als Polyamine in der Komponente **K2** geeignet sind primäre aliphatische Polyamine, wie sie als Amine **B2** der Formel (III b) bereits beschrieben wurden; sekundäre aliphatische Polyamine wie beispielsweise N,N'-Dibutyl-ethylendiamin; N,N'-Di-tert.butyl-ethylendiamin, N,N'-Diethyl-1,6-hexandiamin, 1-(1-Methylethyl-amino)-3-(1-methylethyl-aminomethyl)-3,5,5-trimethylcyclohexan (Jefflink^{®} 754 von Huntsman), N4-Cyclohexyl-2-methyl-N2-(2-methylpropyl)-2,4-pentandiamin, N,N'-Dialkyl-1,3-xylylendiamin, Bis-(4-(N-alkylamino)-cyclohexyl)-methan, N-alkylierte Polyetheramine, beispielsweise die Jeffamine^{®}-Typen SD-231, SD-401, SD-404 und SD-2001 (alle von Huntsman), Produkte aus der Michael-artigen Addition der beispielhaft erwähnten primären aliphatischen Polyamine an Michaelakzeptoren wie Maleinsäurediester, Fumarsäurediester, Citraconsäurediester, Acrylsäureester, Methacrylsäureester, Zimtsäureester, Itaconsäurediester, Vinylphosphonsäurediester, Vinylsulfonsäurearylester, Vinylsulfone, Vinylnitrile, 1-Nitroethylene oder Knoevenagel-Kondensationsprodukte wie beispielsweise solche aus Malonsäurediestern und Aldehyden wie Formaldehyd, Acetaldehyd oder Benzaldehyd; aliphatische Polyamine mit primären und sekundären Aminogruppen, wie beispielsweise N-Butyl-1,6-hexandiamin; primäre und/oder sekundäre aromatische Polyamine wie beispielsweise m- und p-Phenylendiamin, 4,4'-Diaminodiphenylmethan (MDA), 3,3'-Dichloro-4,4'-diaminodiphenylmethan (MOCA), Mischungen von 3,5-Dimethylthio-2,4- und -2,6-toluylendiamin (erhältlich als Ethacure^{®} 300 von Albemarle), Mischungen von 3,5-Diethyl-2,4- und -2,6-toluylendiamin (DETDA), 3,3',5,5'-Tetraethyl-4,4'-diaminodiphenylmethan (M-DEA), 3,3',5,5'-Tetraethyl-2,2'-dichloro-4,4'-diaminodiphenylmethan (M-CDEA), 3,3'-Diisopropyl-5,5'-dimethyl-4,4'-diaminodiphenylmethan (M-MIPA), 3,3',5,5'-Tetraisopropyl-4,4'-diaminodiphenylmethan (M-DIPA), 4,4'-Diaminodiphenylsulfon (DDS), 4-Amino-N-(4-aminophenyl)-benzolsulfonamid, 5,5'-Methylendianthranilsäure, Dimethyl-(5,5'-Methylendianthranilat), 1,3-Propylen-bis-(4-aminobenzoat), 1,4-Butylen-bis-(4-aminobenzoat), Polytetramethylenoxid-bis-(4-aminobenzoat) (erhältlich als Versalink^{®} von Air Products), 1,2-Bis-(2-aminophenylthio)-ethan, N,N'-Dialkyl-p-phenylendiamin, N,N'-Dialkyl-4,4'-diaminodiphenylmethan, 2-Methylpropyl-(4-chloro-3,5-diaminobenzoat) und tert.Butyl-(4-chloro-3,5-diaminobenzoat); sowie Polyamine mit mehr als drei Aminogruppen.

Als Polyole in der Komponente **K2** geeignet sind dieselben Polyole, wie sie bereits als geeignet zur Herstellung eines Polyurethanpolymers **PUP** erwähnt wurden, sowie diejenigen niedrigmolekularen zwei- oder mehrwertigen Alkohole, wie sie vorgängig als geeignet zum Mitverwenden bei der Herstellung eines Polyurethanpolymers **PUP** erwähnt wurden.

Als Aminoalkohole in der Komponente **K2** geeignet sind Verbindungen, welche mindestens eine primäre oder sekundäre Aminogruppe und mindestens eine Hydroxylgruppe aufweisen, wie beispielsweise die aliphatischen Hydroxyamine, welche bereits vorgängig als geeignete Amine **B1** zur Herstellung der Aldimine der Formel (I) erwähnt wurden, sowie weiterhin beispielsweise Diethanolamin, 2-(Methylamino)ethanol, 2-(Ethylamino)ethanol, 2-(Butylamino)ethanol und 2-(Cyclohexylamino)ethanol.

Als Polythiole in der Komponente **K2** geeignet sind beispielsweise unter dem Markennamen Thiokol^{®} bekannte flüssige Mercapto-terminierte Polymere, beispielsweise die Typen LP-3, LP-33, LP-980, LP-23, LP-55, LP-56, LP-12, LP-31, LP-32 und LP-2 (Morton Thiokol; beispielsweise erhältlich von SPI Supplies, USA, oder von Toray Fine Chemicals, Japan), sowie Polyester von Thiocarbonsäuren, beispielsweise Pentaerythritoltetramercaptoacetat, Trimethylolpropantrimercaptoacetat, Glykoldimercaptoacetat, Pentaerythritoltetra-(3-mercaptopropionat), Trimethylolpropantri-(3-mercaptopropionat) und Glykoldi-(3-mercaptopropionat).

Neben den Aldiminen der Formel (I') können weitere blockierte Amine als Bestandteil der Komponente **K2** eingesetzt werden, insbesondere Ketimine, Oxazolidine, Enamine sowie andere Aldimine. Solche anderen Aldimine sind erhältlich ausgehend von anderen Aldehyden als den vorgängig genannten Aldehyden **ALD1** der Formel (IV'), wie beispielsweise nur eine Hydroxylgruppe aufweisende Aldehyde **ALD** der Formel (IV), Isobutyraldehyd, sowie die Produkte aus der Veresterung von Carbonsäuren, wie sie in WO 2004/013088 A1 beschrieben werden, insbesondere die Produkte aus der Veresterung von Laurinsäure mit 3-Hydroxypivalaldehyd. Ketimine sind beispielsweise erhältlich aus der Umsetzung der vorgängig beschriebenen Amine **B** der Formel (III) mit Ketonen. Geeignete Oxazolidine sind insbesondere Polyoxazolidine, wie zum Beispiel Härter OZ (Bayer). Geeignete Enamine sind beispielsweise erhältlich aus der Umsetzung von Aminen mit mehreren sekundären Aminogruppen mit aliphatischen oder cycloaliphatischen Aldehyden oder Ketonen, die am C-Atom in α-Stellung zur Carbonylgruppe mindestens ein Wasserstoffatom aufweisen.

In einer Ausführungsform enthält die Komponente **K2** Wasser. Die Komponente **K2** enthält insbesondere die zur Hydrolyse der Aldiminogruppen und anderen blockierten Aminogruppen benötigte Wassermenge, oder ein Teil davon.

Die besonders bevorzugte härtbare zweikomponentige Zusammensetzung enthält gegebenenfalls weitere Bestandteile, insbesondere in Polyurethanzusammensetzungen üblicherweise eingesetzte Hilfs- und Zusatzstoffe, beispielsweise die folgenden:
- Weichmacher, beispielsweise Carbonsäureester wie Phthalate, beispielsweise Dioctylphthalat, Diisononylphthalat oder Diisodecylphthalat, Adipate, beispielsweise Dioctyladipat, Azelate und Sebacate, organische Phosphor- und Sulfonsäureester oder Polybutene;
- nicht-reaktive thermoplastische Polymere, wie beispielsweise Homo- oder Copolymere von ungesättigten Monomeren, insbesondere aus der Gruppe umfassend Ethylen, Propylen, Butylen, Isobutylen, Isopren, Vinylacetat und Alkyl(meth)acrylate, insbesondere Polyethylene (PE), Polypropylene (PP), Polyisobutylene, Ethylenvinylacetat-Copolymere (EVA) und ataktische Poly-α-Olefine (APAO);
- Lösemittel;
- anorganische und organische Füllstoffe, zum Beispiel gemahlene oder gefällte Calciumcarbonate, welche gegebenenfalls mit Fettsäuren, insbesondere Stearaten, beschichtet sind, Baryt (BaSO₄, auch Schwerspat genannt), Quarzmehle, calcinierte Kaoline, Aluminiumoxide, Aluminiumhydroxide, Kieselsäuren, insbesondere hochdisperse Kieselsäuren aus Pyrolyseprozessen, Russe, insbesondere industriell hergestellte Russe (im Folgenden als "Russ" bezeichnet), PVC-Pulver oder Hohlkugeln;
- Fasern, beispielsweise aus Polyethylen;
- Pigmente, beispielsweise Titandioxid oder Eisenoxide;
- Katalysatoren, welche die Hydrolyse von Aldiminen beschleunigen, insbesondere Säuren, beispielsweise organische Carbonsäuren wie Benzoesäure, Salicylsäure oder 2-Nitrobenzoesäure, organische Carbonsäureanhydride wie Phthalsäureanhydrid, Hexahydrophthalsäureanhydrid und Hexahydromethylphthalsäureanhydrid, Silylester von organischen Carbonsäuren, organische Sulfonsäuren wie Methansulfonsäure, p-Toluolsulfonsäure oder 4-Dodecylbenzolsulfonsäure, Sulfonsäureester, andere organische oder anorganische Säuren, oder Mischungen der vorgenannten Säuren und Säureester;
- Katalysatoren, welche die Reaktion der Isocyanatgruppen beschleunigen, beispielsweise Organozinnverbindungen wie Dibutylzinndiacetat, Dibutylzinndilaurat, Dibutylzinndichlorid, Dibutylzinndiacetylacetonat und Dioctylzinndilaurat, Bismutverbindungen wie Bismuttrioctoat und Bismuttris(neodecanoat), und tertiäre Aminogruppen enthaltende Verbindungen wie 2,2'-Dimorpholinodiethylether und 1,4-Diazabicyclo[2.2.2]octan;
- Rheologie-Modifizierer wie beispielsweise Verdickungsmittel oder Thixotropiermittel, zum Beispiel Harnstoffverbindungen, Polyamidwachse, Bentonite oder pyrogene Kieselsäuren;
- Reaktivverdünner und Vernetzer, beispielsweise monomere Diisocyanate sowie Oligomere und Derivate dieser Polyisocyanate, Addukte monomerer Polyisocyanate mit kurzkettigen Polyolen, sowie Adipinsäuredihydrazid und andere Dihydrazide, sowie Polyisocyanate mit blockierten Isocyanatgruppen, wie sie bereits vorgängig erwähnt wurden;
- blockierte Amine, beispielsweise in Form von Ketiminen, Oxazolidinen, Enaminen oder anderen Aldiminen;
- Trocknungsmittel, wie beispielsweise Molekularsiebe, Calciumoxid, hochreaktive Isocyanate wie p-Tosylisocyanat, Orthoameisensäureester, AIkoxysilane wie Tetraethoxysilan;
- Organoalkoxysilane, im Folgenden auch "Silane" genannt, wie beispielsweise Epoxysilane, (Meth)acrylsilane, Isocyanatosilane, Vinylsilane, Carbamatosilane, Alkylsilane, S-(Alkylcarbonyl)-mercaptosilane und Aldiminosilane, sowie oligomere Formen dieser Silane;
- Stabilisatoren gegen Wärme, Licht- und UV-Strahlung;
- flammhemmende Substanzen;
- oberflächenaktive Substanzen wie beispielsweise Netzmittel, Verlaufsmittel, Entlüftungsmittel oder Entschäumer;
- Biozide wie beispielsweise Algizide, Fungizide oder das Pilzwachstum hemmende Substanzen.

Es ist vorteilhaft, beim Einsatz solcher weiterer Bestandteile darauf zu achten, dass diese die Lagerstabilität der jeweiligen Komponente **K1** oder **K2** der Zusammensetzung nicht stark beeinträchtigen. Falls solche Zusätze als Bestandteil der Komponente **K1** vorhanden sind, ist darauf zu achten, dass sie während der Lagerung die Vernetzung der Isocyanatgruppen nicht in signifikantem Ausmass auslösen. Insbesondere bedeutet dies, dass solchermassen verwendete Zusätze kein oder höchstens Spuren von Wasser enthalten sollten. Es kann sinnvoll sein, gewisse Zusätze vor dem Einmischen in die Komponente **K1** chemisch oder physikalisch zu trocknen.

Im Fall der Komponente **K2** sind neben diesen zusätzlich noch weitere Hilfs- und Zusatzstoffe möglich, welche zusammen mit freien Isocyanatgruppen nicht oder nur kurzzeitig lagerfähig sind. Insbesondere sind dies Katalysatoren wie:
Verbindungen von Zink, Mangan, Eisen, Chrom, Cobalt, Kupfer, Nickel, Molybdän, Blei, Cadmium, Quecksilber, Antimon, Vanadium, Titan, Zirconium oder Kalium, wie Zink(II)-acetat, Zink(II)-2-ethylhexanoat, Zink(II)-laurat, Zink(II)-oleat, Zink(II)-naphthenat, Zink(II)-acetylacetonat, Zink(II)-salicylat, Mangan(II)-2-ethylhexanoat, Eisen(III)-2-ethylhexanoat, Eisen(III)-acetylacetonat, Chrom(III)-2-ethylhexanoat, Cobalt(II)-naphthenat, Cobalt(II)-2-ethylhexanoat, Kupfer(II)-2-ethylhexanoat, Nickel(II)-naphthenat, Phenylquecksilber-neodecanoat, Blei(11)-acetat, Blei(II)-2-ethylhexanoat, Blei(II)-neodecanoat, Blei(II)-acetylacetonat, Aluminiumlactat, Aluminiumoleat, Aluminium(III)-acetylacetonat, Diisopropoxytitan-bis-(ethylacetoacetat), Dibutoxytitan-bis-(ethylacetoacetat), Dibutoxytitan-bis-(acetylacetonat), Kalium-acetat, Kaliumoctoat; tertiäre Amine wie Triethylamin, Tributylamin, N-Ethyl-diisopropylamin, N,N,N',N'-Tetramethyl-ethylendiamin, Pentamethyl-diethylentriamin und höhere Homologe davon, N,N,N',N'-Tetramethyl-propylendiamin, Pentamethyl-dipropylentriamin und höhere Homologe davon, N,N,N',N'-Tetramethyl-1,3-butandiamin, N,N,N',N'-Tetramethyl-1,6-hexandiamin, Bis-(dimethylamino)-methan, N,N-Dimethylbenzylamin, N,N-Dimethylcyclohexylamin, N-Methyl-dicyclohexylamin, N,N-Dimethyl-hexadecylamin, Bis-(N,N-diethylaminoethyl)-adipat, N,N-Dimethyl-2-phenylethylamin, Tris-(3-dimethylaminopropyl)-amin, 1,4-Diazabicyclo[2.2.2]octan (DABCO), 1,8-Diazabicyclo[5.4.0]undec-7-en (DBU), 1,5-Diazabicyclo[4.3.0]non-5-en (DBN), N-Methylmorpholin, N-Ethylmorpholin, N-Cocomorpholin, N,N'-Dimethylpiperazin, N-Methyl-N'-dimethylaminoethyl-piperazin, Bis-(dimethylaminoethyl)-piperazin, 1,3,5-Tris-(dimethylaminopropyl)-hexahydrotriazin, Bis-(2-dimethylaminoethyl)-ether; stickstoffaromatische Verbindungen wie 4-Dimethylamino-pyridin, N-Methylimidazol, N-Vinylimidazol oder 1,2-Dimethylimidazol; Amidine und Guanidine wie 1,1,3,3-Tetramethylguanidin; tertiäre Amine enthaltend aktive Wasserstoffatome, wie Triethanolamin, Triisopropanolamin, N-Methyldiethanolamin, N,N-Dimethylethanolamin, 3-(Dimethylamino)-propyl-diisopropanolamin, Bis-(3-(dimethylamino)-propyl)-isopropanolamin, Bis-(3-dimethylaminopropyl)amin, 3-(Dimethylamino)-propylharnstoff, Mannich-Basen von Phenolen wie 2,4,6-Tris-(dimethylaminomethyl)-phenol oder 2,4,6-Tris-(3-(dimethylamino)-propylaminomethyl)-phenol, Imidazole wie beispielsweise N-Hydroxypropylimidazol oder N-(3-Aminopropyl)-imidazol, sowie Alkoxylierungs- und Polyalkoxylierungsprodukte dieser Verbindungen, beispielsweise Dimethylaminoethoxyethanol; organische Ammoniumverbindungen wie Benzyltrimethylammoniumhydroxid oder alkoxylierte tertiäre Amine; sogenannte "delayed action"-Katalysatoren, welche Modifizierungen bekannter Metall- oder Aminkatalysatoren darstellen, wie Umsetzungsprodukte aus tertiären Aminen und Carbonsäuren oder Phenolen, beispielsweise aus 1,4-Diazabicyclo[2.2.2]octan oder DBU und Ameisensäure oder Essigsäure; sowie Kombinationen der genannten Verbindungen, insbesondere von Metallverbindungen und tertiären Aminen.

Bevorzugt enthält die Zusammensetzung mindestens einen Katalysator in Form einer metallorganischen Verbindung und / oder eines tertiären Amins und / oder einer Säure, insbesondere einer organischen Carbon- oder Sulfonsäure.

Die Herstellung der beiden Komponenten **K1** und **K2** erfolgt getrennt voneinander, für die Komponente **K1** unter Ausschluss von Feuchtigkeit. Die beiden Komponenten **K1** und **K2** sind getrennt voneinander lagerstabil, d.h. sie können jede in einer geeigneten Verpackung oder Anordnung, wie beispielsweise einem Fass, einem Beutel, einem Eimer, einer Kartusche oder einer Flasche, vor ihrer Anwendung während mehreren Monaten bis zu einem Jahr und länger aufbewahrt werden, ohne dass sie sich in ihren jeweiligen Eigenschaften in einem für ihren Gebrauch relevanten Ausmass verändern.

Zur Verwendung der zweikomponentigen Zusammensetzung werden die beiden Komponenten **K1** und **K2** miteinander vermischt. Dabei ist darauf zu achten, dass das Mischungsverhältnis so gewählt wird, dass die gegenüber Isocyanatgruppen reaktiven Bestandteile in einem geeigneten Verhältnis zu den Isocyanatgruppen der Komponente **K1** stehen. Insbesondere beträgt das Verhältnis 0.1 bis 1.1, bevorzugt 0.5 bis 0.95, besonders bevorzugt 0.6 bis 0.9, Equivalent der Summe der vorhandenen Hydroxylgruppen, Aminogruppen, Mercaptogruppen und geschützten Aminogruppen pro Equivalent Isocyanatgruppen, wobei geschützte Aminogruppen in Form von Oxazolidinogruppen doppelt gerechnet werden. Bei der Aushärtung reagieren überschüssige Isocyanatgruppen mit Feuchtigkeit, insbesondere mit Luftfeuchtigkeit.

Das Vermischen der beiden Komponenten **K1** und **K2** erfolgt mit einem geeigneten Verfahren, beispielsweise über einen Statikmischer. Das Vermischen kann kontinuierlich oder batchweise erfolgen. Die vermischte Zusammensetzung wird anschliessend auf ein Substrat appliziert, gegebenenfalls mittels eines geeigneten Applikationshilfsmittels. Dabei muss darauf geachtet werden, dass zwischen dem Vermischen der Komponenten und der Applikation nicht zuviel Zeit vergeht, da durch ein zu starkes Vorreagieren der Bestandteile der vermischten Zusammensetzung vor der Applikation die Funktion der ausgehärteten Zusammensetzung gestört sein kann, beispielsweise indem die Haftung zum Substrat nur ungenügend oder verzögert aufgebaut wird. Die maximale Zeitspanne, innerhalb welcher die vermischte Zusammensetzung appliziert sein sollte, wird als "Topfzeit" bezeichnet.

Nach dem Vermischen der Komponenten **K1** und **K2** beginnt die Aushärtung. Die Aldiminogruppen beginnen in der bereits beschriebenen Weise mit den Isocyanatgruppen zu reagieren, sobald sie mit Wasser in Kontakt kommen. Entweder ist das Wasser in der vermischten Zusammensetzung bereits vorhanden - indem es ein Bestandteil der Komponente **K2** war, oder indem es der Zusammensetzung vor oder während dem Vermischen der beiden Komponenten **K1** und **K2** zugesetzt wurde -, oder das Wasser diffundiert in der Form von Luftfeuchtigkeit in die vermischte Zusammensetzung. Im letztgenannten Fall erfolgt die Reaktion der Aldiminogruppen mit den Isocyanatgruppen von aussen nach innen, parallel zum Eindringen der Feuchtigkeit aus der Luft in die Zusammensetzung. Wie bereits beschrieben, muss die Reaktion der Isocyanatgruppen mit den hydrolysierenden Aldiminogruppen nicht notwendigerweise über freie Aminogruppen erfolgen, sondern kann auch über Zwischenstufen der Hydrolysereaktion erfolgen. Auf die gleiche Weise werden die Reaktivgruppen von weiteren gegebenenfalls in der Zusammensetzung vorhandenen blockierten Aminen freigesetzt. Weiterhin reagieren nach dem Vermischen der Komponenten **K1** und **K2** die in der Zusammensetzung vorhandenen Hydroxyl-, Mercapto- und Aminogruppen mit den Isocyanatgruppen. Die an den Resten R^{4'} und R^{5'} vorhandenen Hydroxylgruppen können entweder vor, während oder nach der formalen Freisetzung des Aldehyds **ALD1** mit den Isocyanatgruppen reagieren. Als Ergebnis dieser Reaktionen vernetzt die vermischte Zusammensetzung und härtet schliesslich zu einem festen Material aus.

Die Aushärtung der beschriebenen härtbaren Zusammensetzungen erfolgt im Allgemeinen ohne die Bildung von Blasen, auch bei hoher Aushärtungsgeschwindigkeit. Die Aushärtungsgeschwindigkeit lässt sich über die Art und Menge eines oder mehrerer gegebenenfalls vorhandener Katalysatoren, über die bei der Aushärtung herrschende Temperatur sowie über die Luftfeuchtigkeit bzw. die Menge zugesetzten Wassers beeinflussen.

Die beschriebenen härtbaren Zusammensetzungen weisen eine Reihe von Vorteilen auf.

Die Aldimine der Formel (I') enthalten sterisch gehinderte, nicht zu Enaminogruppen tautomerisierbare Aldiminogruppen. Diese reagieren in Anwesenheit von Wasser mit Isocyanatgruppen formal als primäre Aminogruppen, wobei ihre Reaktivität im Vergleich mit den entsprechenden freien primären Aminogruppen stark vermindert ist, so dass solche Systeme eine gut handhabbare Aushärtungsgeschwindigkeit aufweisen.

Die Anwesenheit der Aldimine verhindert die direkte, Kohlendioxid (CO₂) erzeugende Reaktion der Isocyanatgruppen mit Feuchtigkeit, welche sich bereits in der Zusammensetzung befindet oder nach der Applikation in die Zusammensetzung gelangt, und unterdrückt damit die Entstehung von unerwünschten Gasblasen beim Aushärten der Zusammensetzung weitgehend.

Weiterhin wirken die bei der Aushärtung aus den Aldiminen der Formel (I') freigesetzten Aldehyde **ALD1** der Formel (IV') ihrerseits als Härter, da sie an den Resten R^{4'} und R^{5'} zusammen mindestens zwei Hydroxylgruppen tragen und deshalb mit den Isocyanatgruppen unter Kettenverlängerung oder Vernetzung des entstehenden Polyurethanpolymers reagieren und nicht zu Kettenabbrüchen führen. Der auf diese Weise erfolgende kovalente Einbau der Aldehyde **ALD1** in das Polymer ist besonders wertvoll, da er die Probleme, welche nicht einbaubare Aldehyde in der ausgehärteten Zusammensetzung verursachen können, wie insbesondere Schwund, unangenehme Gerüche, Ausschwitzungen oder Verminderung mechanischer Festigkeit und Beständigkeit, verhindert.

Ferner können die Aldimine der Formel (I') aufgrund ihres Gehalts an tertiären Aminogruppen eine katalytische Wirkung auf die Reaktion der Isocyanatgruppen ausüben und so die Aushärtung beschleunigen. Diese beschleunigende Wirkung wird zusätzlich unterstützt durch die Tatsache, dass die tertiären Aminogruppen im Aldehyd-Teil der Aldimine, d.h. nach deren Hydrolyse in den freigesetzten Aldehyden **ALD1,** lokalisiert sind. Es ist dabei aber vorteilhaft, dass die Basizität dieser tertiären Aminogruppen vergleichsweise niedrig ist, da stark basische tertiäre Amine die säurekatalysierte Hydrolyse der Aldiminogruppen stören und/oder die direkte Reaktion der Isocyanatgruppen, insbesondere mit Wasser, übermässig beschleunigen können, was eine unvollständige Aushärtung zur Folge haben kann. Die tertiären Aminogruppen sind im Aldehyd-Teil der Aldimine der Formel (I') lokalisiert und werden bei der Aushärtung über die Reaktion der Hydroxylgruppen mit Isocyanatgruppen kovalent in das entstehende Polymer eingebaut. Nach dem Einbau ins Polymer ist die katalytische Aktivität der tertiären Aminogruppe aufgrund deren eingeschränkten Beweglichkeit wesentlich reduziert, was für die Beständigkeit des Materials von Vorteil sein kann.

Die bei der Hydrolyse der Aldiminogruppen entstehenden Aldehydgruppen bleiben bei der Aushärtung erhalten und werden über die beschriebenen Reaktionen kovalent in das entstehende Polymer eingebaut. Sie können, falls gewünscht, für weiterführende Reaktionen verwendet werden.

Ein weiterer Vorteil der beschriebenen Zusammensetzungen liegt im vergleichsweise geringen Geruch der beschriebenen Aldimine der Formel (I') und der Aldehyde **ALD1.** Dadurch weisen die Zusammensetzungen vor, während und nach der Aushärtung keinen oder nur einen geringen Geruch auf.

Bevorzugte Anwendungen der beschriebenen härtbaren Zusammensetzungen sind Klebstoffe, Dichtstoffe, Vergussmassen, Beschichtungen, Bodenbeläge, Anstriche, Lacke, Primer und Schäume. Einzelne Anwendungen sollen im Weiteren kurz beschrieben werden, was jedoch eine anderweitige Anwendung dieser Zusammensetzungen keineswegs einschränken soll.

In einer bevorzugten Ausführungsform wird eine der beschriebenen härtbaren Zusammensetzungen als Kleb- oder Dichtstoff verwendet. In dieser Anwendung enthält die härtbare Zusammensetzung vorteilhaft mindestens einen Füllstoff, wobei dieser sowohl die rheologischen Eigenschaften der nicht ausgehärteten Zusammensetzung als auch die mechanischen Eigenschaften und die Oberflächenbeschaffenheit der ausgehärteten Zusammensetzung beeinflusst. Geeignete Füllstoffe sind die bereits genannten anorganischen und organischen Füllstoffe. Bevorzugt sind Russ,Calciumcarbonate, calcinierte Kaoline, hochdisperse Kieselsäuren, PVC-Pulver sowie flammhemmende Füllstoffe wie Hydrate oder Hydroxide, insbesondere Aluminiumhydroxid. Der Gehalt an Füllstoff liegt insbesondere im Bereich von 10 bis 70 Gewichts-%, bevorzugt von 20 bis 60 Gewichts-%, bezogen auf die gesamte Zusammensetzung. Es kann von Vorteil sein, eine Mischung verschiedener Füllstoffe einzusetzen.

Weiterhin enthält die härtbare Zusammensetzung in der Anwendung als Kleb- oder Dichtstoff vorteilhaft mindestens einen der bereits genannten Katalysatoren, welcher die Hydrolyse der Aldiminogruppen beziehungsweise die Reaktion der Isocyanatgruppen beschleunigt. Insbesondere geeignet sind Gemische aus organischen Säuren und einer metallorganischen Verbindung oder einem Metallkomplex, aus einer organischen Säure und einer tertiäre Aminogruppen enthaltenden Verbindung, oder Gemische aus organischen Säuren, einer metallorganischen Verbindung oder einem Metallkomplex, und einer tertiäre Aminogruppen enthaltenden Verbindung. Ein typischer Gehalt an Katalysatoren beträgt 0.005 bis 2 Gewichts-% bezogen auf die gesamte Zusammensetzung, wobei dem Fachmann klar ist, welche Einsatzmengen für welche Katalysatoren sinnvoll sind.

Ein Kleb- oder Dichtstoff wird in der bereits beschriebenen Weise hergestellt und appliziert.

Geeignete Anwendungen eines Klebstoffes sind beispielsweise das Verkleben von Bauteilen im Hoch- oder Tiefbau und bei der Fertigung oder Reparatur von industriellen Gütern oder Konsumgütern, insbesondere von Fenstern, Haushaltmaschinen oder Transportmitteln wie Fahrzeugen zu Wasser oder zu Lande, bevorzugt Automobile, Busse, Lastkraftwagen, Züge oder Schiffe, sowie das Verkleben von Artikeln der Möbel-, Textil- oder Verpackungsindustrie; oder das Abdichten von Fugen, Nähten oder Hohlräumen in der industriellen Fertigung oder Reparatur, oder im Hoch- oder Tiefbau.

Geeignete Anwendungen eines Dichtstoffes sind beispielsweise das Abdichten eines Bauwerkes, insbesondere Fugen im Hoch- oder Tiefbau, oder das Abdichten eines Teils eines Bauwerkes, beispielsweise eines Fensters oder eines Fussbodens, oder das Abdichten eines industriellen Gutes, wie beispielsweise einer Haushaltmaschine oder eines Transportmittels, insbesondere eines Fahrzeugs zu Wasser oder zu Lande, oder eines Teils davon.

Der Kleb- oder Dichtstoff weist im ausgehärteten Zustand typischerweise elastische Eigenschaften auf.

In einer weiteren bevorzugten Ausführungsform wird eine der beschriebenen härtbaren Zusammensetzungen als Beschichtung verwendet. In dieser Anwendung enthält die härtbare Zusammensetzung vorteilhaft mindestens einen Füllstoff, wobei dieser sowohl die rheologischen Eigenschaften der nicht ausgehärteten Zusammensetzung als auch die mechanischen Eigenschaften und die Oberflächenbeschaffenheit der ausgehärteten Zusammensetzung beeinflusst. Geeignet sind die bereits genannten anorganischen und organischen Füllstoffe. Bevorzugt sind Calciumcarbonate, Baryt und Quarzmehle, sowie flammhemmende Füllstoffe wie Hydrate oder Hydroxide, insbesondere Aluminiumhydroxid. Der Gehalt an Füllstoff liegt insbesondere im Bereich von 10 bis 70 Gewichts-%, bevorzugt von 20 bis 60 Gewichts-%, bezogen auf die gesamte Zusammensetzung. Es kann von Vorteil sein, eine Mischung verschiedener Füllstoffe einzusetzen.

Weiterhin enthält die härtbare Zusammensetzung in der Anwendung als Beschichtung vorteilhaft mindestens einen Katalysator. Dabei sind dieselben Katalysatoren in denselben Mengen geeignet, wie sie bereits als geeignete Bestandteile von Kleb- und Dichtstoffe erwähnt wurden.

Weiterhin enthält die härtbare Zusammensetzung in der Anwendung als Beschichtung vorteilhaft mindestens einen weiteren der bereits genannten Hilfs- und Zusatzstoffe, insbesondere ausgewählt aus der Gruppe umfassend Pigmente, Lösemittel, Verlaufsmittel, Entschäumer und Stabilisatoren.

Geeignete Lösemittel sind beispielsweise Ketone wie Aceton, Methylethylketon, Methylisobutylketon, Diisobutylketon und Mesityloxid, sowie cyclische Ketone wie Cyclohexanon und Methylcyclohexanon; Ester wie Methylacetat, Ethylacetat, Propylacetat, Butylacetat, tert.Butylacetat, Formiate, Propionate oder Malonate; Ether wie Ketonether, Esterether und Dialkylether wie Diisopropylether, Diethylether, Dibutylether, Methyl-tert.butylether, Diethylenglykoldiethylether sowie Ethylenglykoldiethylether; aliphatische und aromatische Kohlenwasserstoffe wie Toluol, Xylol, Heptan, Octan, und Erdölfraktionen wie Naphtha, White Spirit, Petrolether oder Benzin; halogenierte Kohlenwasserstoffe wie Methylenchlorid; sowie N-alkylierte Lactame wie beispielsweise N-Methylpyrrolidon, N-Cyclohexylpyrrolidon oder N-Dodecylpyrrolidon. Der Gehalt an Lösemitteln liegt insbesondere im Bereich von 0 bis 30 Gewichts-%, bevorzugt 0 bis 20 Gewichts-%, bezogen auf die gesamte Zusammensetzung.

Eine Beschichtung wird in der bereits beschriebenen Weise hergestellt und appliziert. Vorteilhaft weist sie eine flüssige Konsistenz mit guten Verlaufseigenschaften auf. Dadurch lässt sie sich einfach als selbstverlaufende Beschichtung auf überwiegend ebene Flächen applizieren, beispielsweise als Bodenbelag. Die beiden Komponenten **K1** und **K2** werden vor der Applikation auf eine geeignete Weise miteinander vermischt und die vermischte Zusammensetzung innerhalb der Topfzeit appliziert.

Die Applikation der härtbaren Zusammensetzung in Form einer Beschichtung erfolgt typischerweise durch Aufgiessen auf den zu beschichtenden Untergrund und wird im flüssigen Zustand mit Hilfe beispielsweise eines Rakels oder einer Zahntraufel gleichmässig verteilt. Zusätzlich kann das Material mit einer Stachelwalze egalisiert und entlüftet werden. Es ist aber auch eine maschinelle Applikation, beispielsweise in Form einer Spritzapplikation, möglich.

Ein geeigneter Untergrund, auf welchen die Zusammensetzung typischerweise appliziert wird, ist beispielsweise Beton, Zement, Asphalt, Stahl, Holz, Keramik oder ein Kunststoff, wobei der Untergrund durch Reinigen, Bürsten oder Sandstrahlen vorbehandelt sein kann, und/oder eine Grundierung aufweisen kann. Als Grundierungen in Frage kommen beispielsweise Haftvermittlerlösungen oder Primer.

Unter einem "Primer" wird im vorliegenden Dokument eine als Voranstrich geeignete Zusammensetzung verstanden, die neben nichtreaktiven flüchtigen Stoffen und optional festen Zuschlägen mindestens ein Polymer und/oder mindestens eine Substanz mit reaktiven Gruppen enthält und die befähigt ist, bei der Applikation auf einem Substrat zu einem festen, gut haftenden Film in einer Schichtdicke von typischerweise mindestens 5 µm auszuhärten, wobei die Aushärtung entweder allein durch das Verdampfen der nichtreaktiven flüchtigen Stoffe, wie beispielsweise Lösemittel oder Wasser, oder durch eine chemische Reaktion, oder durch eine Kombination dieser Faktoren, zustande kommt, und welche eine gute Haftung zu einer nachfolgend aufgebrachten Schicht, beispielsweise einem Kleb- oder Dichtstoff, aufbaut.

Bei einem fertigen Bodenbelag handelt es sich häufig um einen Aufbau aus mehreren sich unterscheidenden Schichten. Ein typischer Aufbau kann beispielsweise mit einer sogenannten Grundierung beginnen, welche die Aufgabe hat, den Untergrund für die Polyurethanbeschichtung vorzubereiten. Anschliessend wird beispielsweise die beschriebene Zusammensetzung, welche im ausgehärteten Zustand typischerweise elastische Eigenschaften aufweist, aufgetragen, wobei dieser Auftrag je nach Beschaffenheit des Untergrundes und gewünschter Schichtdicke in einem oder mehreren Arbeitsgängen erfolgen kann. Pro Schicht wird üblicherweise eine Schichtdicke von 0.5 bis 3 mm, insbesondere 0.5 bis 2 mm, appliziert. Schliesslich kann anschliessend eine sogenannte Versiegelung aufgetragen werden, welche in einer dünnen Schicht, beispielsweise in einer Dicke von einigen Mikrometern bis einigen Zehntelmillimetern, die Oberflächenbeschaffenheit des Bodenbelages nochmals beeinflusst. Dabei kann es sich um eine transparente oder um eine pigmentierte Versiegelung handeln.

Die beschriebene Beschichtung kann vorteilhaft verwendet werden im Innen- oder Aussenbereich eines Gebäudes oder eines Bauwerks, beispielsweise als Bodenbelag für Innenräume wie Büros, Industriehallen, Turnhallen oder Kühlräume oder im Aussenbereich für Balkone, Terrassen, Brücken, Parkdecks oder Sport- und Spielplätze.

Ein weiterer Aspekt der vorliegenden Erfindung betrifft ein Verfahren zum Verkleben von einem Substrat **S1** mit einem Substrat **S2,** welches die Schritte umfasst:
i) Applikation einer vorgängig beschriebenen härtbaren Zusammensetzung auf ein Substrat **S1;**
ii) Kontaktieren der applizierten Zusammensetzung mit einem Substrat **S2** innerhalb der Offenzeit der Zusammensetzung;
   oder
i') Applikation einer vorgängig beschriebenen Zusammensetzung auf ein Substrat **S1** und auf ein Substrat **S2;**
ii') Kontaktieren der applizierten Zusammensetzung miteinander innerhalb der Offenzeit der Zusammensetzung;
wobei das Substrat **S2** aus dem gleichen oder einem unterschiedlichen Material wie das Substrat **S1** besteht.

Ein weiterer Aspekt der vorliegenden Erfindung betrifft ein Verfahren zum Abdichten. Dieses umfasst den Schritt:
i") Applikation einer vorgängig beschriebenen härtbaren Zusammensetzung zwischen ein Substrat **S1** und ein Substrat **S2,** so dass die Zusammensetzung mit dem Substrat **S1** und dem Substrat **S2** in Kontakt steht;
wobei das Substrat **S2** aus dem gleichen oder einem unterschiedlichen Material wie das Substrat **S1** besteht.

Üblicherweise wird der Dichtstoff in eine sogenannte Fuge eingepresst.

Ein weiterer Aspekt der vorliegenden Erfindung betrifft ein Verfahren zum Beschichten eines Substrates **S1.** Dieses umfasst den Schritt:
i''') Applikation einer vorgängig beschriebenen härtbaren Zusammensetzung auf ein Substrat **S1** innerhalb der Offenzeit der Zusammensetzung.

In diesen drei Verfahren sind geeignete Substrate **S1** und/oder **S2** beispielsweise anorganische Substrate wie Glas, Glaskeramik, Beton, Mörtel, Backstein, Ziegel, Gips und Natursteine wie Granit oder Marmor; Metalle oder Legierungen wie Aluminium, Stahl, Buntmetalle, verzinkte Metalle; organische Substrate wie Leder, Stoffe, Papier, Holz, mit Harz gebundene Holzwerkstoffe, Harz-Texil-Compositewerkstoffe, Kunststoffe wie Polyvinylchlorid (Hart- und Weich-PVC), Acrylonitril-Butadien-Styrol-Copolymere (ABS), SMC (Sheet Moulding Composites), Polycarbonat (PC), Polyamid (PA), Polyester, PMMA, Polyester, Epoxidharze, Polyurethane (PUR), Polyoxymethylen (POM), Polyolefine (PO), insbesondere mittels Plasma, Corona oder Flammen oberflächenbehandeltes Polyethylen (PE) oder Polypropylen (PP), Ethylen/Propylen-Copolymere (EPM) und Ethylen/Propylen-Dien-Terpolymere (EPDM); beschichtete Substrate wie pulverbeschichtete Metalle oder Legierungen; sowie Farben und Lacke, insbesondere Automobillacke.

Die Substrate können bei Bedarf vor dem Applizieren der Zusammensetzung vorbehandelt werden. Derartige Vorbehandlungen umfassen insbesondere physikalische und/oder chemische Reinigungsverfahren, beispielsweise Schleifen, Sandstrahlen, Bürsten oder dergleichen, oder Behandeln mit Reinigern oder Lösemitteln oder das Aufbringen eines Haftvermittlers, einer Haftvermittlerlösung oder eines Primers.

Im Fall einer zweikomponentigen Zusammensetzung werden die beiden Komponenten **K1** und **K2** kurz vor der Applikation miteinander vermischt.

Im Fall einer hitzehärtenden Zusammensetzung wird die applizierte Zusammensetzung anschliessend an die Verklebung, die Abdichtung oder die Beschichtung eingebrannt, indem sie auf eine geeignete Temperatur erhitzt wird.

Die Applikation der härtbaren Zusammensetzung kann in einem breiten Temperaturspektrum erfolgen. Beispielsweise kann die Zusammensetzung bei Raumtemperatur appliziert werden, wie es für einen Klebstoff oder einen Dichtstoff typisch ist. Die Zusammensetzung kann aber auch bei tieferen wie auch bei höheren Temperaturen appliziert werden. Letzteres ist insbesondere dann vorteilhaft, wenn die Zusammensetzung hochviskose oder schmelzbare Komponenten enthält, wie sie in Schmelzklebstoffen, beispielsweise Warmschmelzklebstoffen (Warm-Melt) oder Heissschmelzklebstoffen (Hot-Melt) typischerweise vorhanden sind. Die Applikationstemperaturen liegen für Warm-Melts beispielsweise im Bereich von 40 bis 80 °C, bei Hot-Melts im Bereich von 85 bis 200 °C.

Aus diesen beschriebenen Verfahren zum Verkleben, Abdichten bzw. Beschichten - beziehungsweise aus der Verwendung einer der beschriebenen Zusammensetzungen als Klebstoff, Dichtstoff, Vergussmasse, Beschichtung, Bodenbelag, Anstrich, Lack, Primer oder Schaum - entsteht ein Artikel.

Dieser Artikel ist insbesondere ein Bauwerk, insbesondere ein Bauwerk des Hoch- oder Tiefbaus, oder ein industrielles Gut oder ein Konsumgut, insbesondere ein Fenster, eine Haushaltmaschine, oder ein Transportmittel, insbesondere ein Fahrzeug zu Wasser oder zu Lande, bevorzugt ein Automobil, ein Bus, ein Lastkraftwagen, ein Zug oder ein Schiff, oder ein Anbauteil eines Transportmittels, oder ein Artikel der Möbel-, Textil-oder Verpackungsindustrie.

### Beispiele

### 1. Beschreibung der Messmethoden

Die **Viskosität** wurde auf einem thermostatisierten Kegel-Platten-Viskosimeter Physica UM (Kegeldurchmesser 20 mm, Kegelwinkel 1 °, Kegelspitze-Platten-Abstand 0.05 mm, Schergeschwindigkeit 10 bis 1000 s⁻¹) gemessen.

Der **Amingehalt,** das heisst der totale Gehalt an freien Aminogruppen und blockierten Aminogruppen (Aldiminogruppen) in den hergestellten Verbindungen, wurde titrimetrisch bestimmt (mit 0.1N HClO₄ in Eisessig, gegen Kristallviolett) und ist stets angegeben in mmol N/g.

Der ***p*Kₐ-Wert** für die konjugierte Säure einer Mannich-Base wurde näherungsweise anhand des Halbneutralisations-Potentials bei der potentiometrischen Titration von ca. 1 mmol Mannich-Base in 50 ml Wasser mit 0.1N HCl bestimmt.

**Infrarotspektren** wurden auf einem FT-IR Gerät 1600 von Perkin-Elmer als unverdünnte Filme auf einer horizontalen ATR-Messeinheit mit ZnSe-Kristall gemessen; die Absorptionsbanden sind in Wellenzahlen (cm⁻¹) angegeben (Messfenster: 4000-650 cm⁻¹); der Zusatz sh weist auf eine als Schulter erscheinende Bande hin, der Zusatz br auf eine breite Bande.

**GC/MS** wurde unter folgenden Bedingungen durchgeführt: Säule Optima-5-MS, 30mx0.25mm, 0.5 µm Filmdicke; Aufheizrate 15 °C/min von 60 °C auf 320 °C, dann 15 min. bei 320 °C gehalten; Trägergas He, 14 psi; Split 15 ml/min; lonisationsmethode EI⁺. Für das Gaschromatogramm ist die Retentionszeit des Produktsignals (t_{R}) angegeben. Beim Massenspektrum werden nur die grössten Peaks angegeben (als m/z); die relative Intensität (in %) und, wenn möglich, eine tentative Zuordnung des Molekülfragments stehen in Klammern.

### 2. Herstellung von Aldehyden

### 3-(N-Bis(2-hydroxyethyl)amino)-2,2-dimethyl-propanal

In einem Rundkolben wurden unter Stickstoffatmosphäre 83.4 g (1.00 mol) 36%iger wässriger Formaldehyd und 75.7 g (1.05 mol) Isobutyraldehyd vorgelegt. Unter gutem Rühren und Eiskühlung wurden aus einem Eintropftrichter langsam 105.1 g (1.00 mol) Diethanolamin zugetropft, wobei darauf geachtet wurde, dass die Temperatur der Reaktionsmischung nicht über 20 °C anstieg. Nach erfolgter Zugabe liess man eine Stunde bei Raumtemperatur rühren. Die entstandene klare, farblose Reaktionsmischung wurde im Ölbad bei 80 °C während 2 Stunden unter Rückfluss gerührt, auf Raumtemperatur abgekühlt und die flüchtigen Bestandteile im Wasserstrahlvakuum bei 80 °C abdestilliert. Man erhielt 181.2 g (96% der Theorie) Rohprodukt als klares, gelbliches Öl, das einen Amingehalt von 5.40 mmol N/g und eine Viskosität von 23.7 Pa.s bei 20 °C aufwies. Das Rohprodukt enthielt neben 3-(N-Bis(2-hydroxyethyl)amino)-2,2-dimethyl-propanal kleinere Anteile von 3-Hydroxy-2,2-dimethyl-propanal, N-(2-Hydroxyethyl)-oxazolidin und N-(2-Hydroxyethyl)-2-isopropyl-oxazolidin (nach GC/MS-Untersuchung).
*p*Kₐ ≈ 7.1.

IR: 3358br(OH),2950,2929sh,2913,2870, 2830, 2719sh br (CHO), 1721 (C=O), 1464, 1391, 1359, 1302br, 1206, 1147, 1078sh, 1037, 966, 940, 920, 883, 786. GC/MS: t_{R} = 10.3 min; Massenspektrum: 189 (2, [M]⁺), 172 (3, [M-OH]⁺), 158 (11, [M-CH₂OH]⁺), 128 (4), 118 (100, [M-C(CH₃)₂CHO]⁺), 116 (15), 102 (6), 98 (5), 88 (2, [118-CHOH]⁺), 88 (72), 86 (21), 74 (50), 56 (51).

### 3-(N-Bis(2-hydroxy-2-methylethyl)amino)-2,2-dimethyl-propanal

Unter gleichen Bedingungen wie vorgängig für die Herstellung von 3-(N-Bis(2-hydroxyethyl)amino)-2,2-dimethyl-propanal beschrieben wurden 83.4 g (1.00 mol) 36%iger wässriger Formaldehyd mit 75.7 g (1.05 mol) Isobutyraldehyd und 133.2 g (1.00 mol) Diisopropanolamin umgesetzt und aufgearbeitet. Man erhielt 199.4 g (92% der Theorie) Rohprodukt als klares, gelbliches Öl, das einen Amingehalt von 4.87 mmol N/g und eine Viskosität von 8.2 Pa.s bei 20 °C aufwies. Das Rohprodukt enthielt neben 3-(N-Bis(2-hydroxy-2-methylethyl)amino)-2,2-dimethyl-propanal kleinere Anteile von 3-Hydroxy-2,2-dimethyl-propanal, N-(2-Hydroxy-2-methylethyl)-oxazolidin und N-(2-Hydroxy-2-methylethyl)-2-isopropyl-oxazolidin (nach GC/MS-Untersuchung). pKₐ ≈ 7.1.
IR: 3392br (OH), 2966, 2933, 2872, 2818, 2719sh br (CHO), 1722 (C=O), 1461, 1409, 1375, 1328, 1274, 1209, 1158, 1130, 1090sh, 1055, 1028sh, 978, 945, 914, 891, 864, 839, 818, 786.
GC/MS: t_{R} = 10.3 min; Massenspektrum: 217 (3, [M]⁺), 172 (30, [M-CH(CH₃)OH]⁺), 146 (44, [M-C(CH₃)₂CHO]⁺), 144 (21), 130 (6), 126 (6), 116 (7), 114 (10), 102 (100, [146-C(CH₃)OH]⁺), 100 (18), 88 (16), 70 (38).

### 3. Herstellung von Aldiminen

### Beispiel 1: Aldimin A-1

In einem Rundkolben wurden unter Stickstoffatmosphäre 68.2 g Polyetherdiamin (Polyoxypropylen-Diamin mit einem mittleren Molekulargewicht von ca. 240 g/mol; Jeffamine^{®} D-230, Huntsman; Amingehalt 8.29 mmol N/g) und 117.6 g 3-(N-Bis(2-hydroxyethyl)amino)-2,2-dimethyl-propanal eingewogen und die Mischung während einer Stunde bei Raumtemperatur gerührt. Danach wurden die flüchtigen Bestandteile im Vakuum entfernt (10 mbar, 80 °C). Ausbeute: 177.1 g eines klaren, gelben Öls mit einem Amingehalt von 6.78 mmol N/g und einer Viskosität von 9.8 Pa.s bei 20 °C.
IR: 3391br (OH), 2964, 2926, 2868, 1662 (C=N), 1469, 1456sh, 1392sh, 1373, 1294, 1106sh, 1049, 1004sh, 926, 903, 877.

### Beispiel 2: Aldimin A-2

Unter gleichen Bedingungen, wie in Beispiel 1 beschrieben, wurden 27.2 g Isophorondiamin (Vestamin^{®} IPD, Degussa; Amingehalt 11.67 mmol N/g) und 71.8 g 3-(N-Bis(2-hydroxy-2-methylethyl)amino)-2,2-dimethyl-propanal umgesetzt. Ausbeute: 93.2 g eines klaren, gelben Honigs mit einem Amingehalt von 7.66 mmol N/g und einer Viskosität von 150 Pa.s bei 20 °C.
IR: 3393br (OH), 2962, 2926, 2898, 2868, 2837, 2818, 1662 (C=N), 1459, 1408, 1373, 1364, 1333, 1273, 1159, 1133, 1116sh, 1058, 1003, 976sh, 945, 909, 891sh, 864, 838.

### Beispiel 3: Aldimin A-3

Unter gleichen Bedingungen, wie in Beispiel 1 beschrieben, wurden 37.7 g Polyetherdiamin (Polyoxypropylen-Diamin mit einem mittleren Molekulargewicht von ca. 240 g/mol; Jeffamine^{®} D-230, Huntsman; Amingehalt 8.29 mmol N/g) und 70.6 g 3-(N-Bis(2-hydroxy-2-methylethyl)amino)-2,2-dimethyl-propanal umgesetzt. Ausbeute: 103.4 g eines klaren, gelb-bräunlichen Öls mit einem Amingehalt von 6.26 mmol N/g und einer Viskosität von 4.0 Pa.s bei 20 °C.
IR: 3419br (OH), 2965, 2925, 2918, 2868, 2822sh, 1662 (C=N), 1457, 1408sh, 1373,1331,1274,1196sh,1106,1089,1059,1019, 1002, 977, 944, 910, 865, 838.

### Beispiel 4: Aldimin A-4

Unter gleichen Bedingungen, wie in Beispiel 1 beschrieben, wurden 6.55 g 2-(2-Aminoethoxy)-ethanol (DGA; Diglycolamine^{®} Agent, Huntsman; Amingehalt 9.39 mmol N/g) und 13.36 g 3-(N-Bis(2-hydroxyethyl)amino)-2,2-dimethyl-propanal umgesetzt. Ausbeute: 16.25 g eines klaren, gelben Öls mit einem Amingehalt von 7.18 mmol N/g und einer Viskosität von 3.4 Pa.s bei 20 °C.
IR: 3358br (OH), 2928, 2865, 2716sh, 1943br, 1663 (C=N), 1467, 1459, 1391, 1358, 1285, 1238, 1123, 1044, 1003sh, 940sh, 924sh, 890, 815, 785, 770.

### Vergleichsbeispiel 5: Aldimin A-5

In einem Rundkolben wurden unter Stickstoffatmosphäre 74.3 g (0.26 mol) destilliertes 2,2-Dimethyl-3-lauroyloxy-propanal vorgelegt. Unter kräftigem Rühren wurden aus einem beheizten Eintropftrichter 30.0 g (0.25 mol N) Polyetherdiamin (Polyoxypropylen-Diamin mit einem mittleren Molekulargewicht von ca. 240 g/mol; Jeffamine^{®} D-230, Huntsman; Amingehalt 8.29 mmol N/g) langsam zugegeben, wobei sich die Mischung erwärmte und zunehmend eintrübte. Danach wurden die flüchtigen Bestandteile im Vakuum entfernt (10 mbar, 80 °C). Ausbeute: 99.5 g eines klaren, blassgelben Öls mit einem Amingehalt von 2.50 mmol N/g.

### 4. Herstellung von härtbaren Zusammensetzungen

### Beispiele 6 bis 9 und Vergleichsbeispiele 10 und 11: 2K-Vergussmassen

Für jedes Beispiel wurden die jeweiligen Bestandteile der Komponente ***K2*** gemäss Tabelle 1 in den angegebenen Gewichtsteilen ohne vorherige Trocknung in einen Polypropylenbecher mit Schraubverschluss eingewogen und mittels eines Zentrifugalmischers (SpeedMixer™ DAC 150, FlackTek Inc.; 2 min. bei 3000 U/min) zu einer homogenen Creme gemischt. Dazu wurden die in der Tabelle 1 angegebenen Gewichtsteile an PMDI als Komponente ***K1*** zugegeben und eingemischt (30 sec. bei 3000 U/min). Das Verhältnis zwischen den Isocyanatgruppen der Komponente ***K1*** und der Summe der Reaktivgruppen (Hydroxyl- und Aldiminogruppen) der Komponente ***K2*** beträgt stets 1.1.

**Tabelle 1: Zusammensetzung der zweikomponentigen Vergussmassen.**

| Beispiel | **6** | **7** | **8** | **9** | **10** **(Vgl)** | **11** **(Vgl)** |
|---|---|---|---|---|---|---|
| **Komponente *K1*:** | | | | | | |
| PMDI^{a} | 35.0 | 35.4 | 34.4 | 38.0 | 28.9 | 29.7 |

| **Komponente *K2*:** | | | | | | |
|---|---|---|---|---|---|---|
| Ricinusöl^{b} | 22.5 | 22.5 | 22.5 | 22.5 | 22.5 | 22.5 |
| Dimerfettsäurediol^{c} | 17.5 | 17.5 | 17.5 | 17.5 | 17.5 | 22.5 |
| Triol^{d} | 4.75 | 4.75 | 4.75 | 4.75 | 4.75 | 4.75 |
| Aldimin | ***A-1,*** 5.0 | ***A-2,*** 5.0 | ***A-3*** 5.0 | ***A-4,*** 5.0 | ***A-5*,** 5.0 | - |
| Säurekatalysator^{e} | 0.25 | 0.25 | 0.25 | 0.25 | 0.25 | 0.25 |
| Kreide^{f} | 50.0 | 50.0 | 50.0 | 50.0 | 50.0 | 50.0 |

| | | | | | | |
|---|---|---|---|---|---|---|
| ^{a} Desmodur^{®} VKS 20 F, Bayer; NCO-Gehalt = 30.0 Gew.-%. ^{b} OH-Zahl = 165 mg KOH/g. ^{c} Sovermol^{®} 908, Cognis ; OH-Zahl = 200 mg KOH/g. ^{d} Desmophen^{®} 4011 T, Bayer; OH-Zahl = 550 mg KOH/g. ^{e} Salicylsäure (5 Gew.-% in Dioctyladipat). ^{f} Omyacarb^{®} 5-GU, Omya. | | | | | | |

Die so erhaltenen Vergussmassen wurden auf Aushärtegeschwindigkeit, mechanische Eigenschaften und Blasenbildung geprüft.

Hinweise zur Aushärtegeschwindigkeit wurden zum einen durch Messen der **Zeit bis zur Klebefreiheit** (tack-free time) erhalten. Dazu wurde ein kleiner Teil der Zusammensetzung unmittelbar nach dem Vermischen in einer Schichtdicke von ca. 2 mm auf Pappkarton aufgetragen und im Normklima (23±1 °C, 50±5% relative Luftfeuchtigkeit) die Zeit bestimmt, die es dauerte, bis beim leichten Antippen der Oberfläche der Zusammensetzung mittels einer Pipette aus LDPE erstmals keine Rückstände auf der Pipette mehr zurückblieben. Zum anderen wurde der weitere Verlauf der Aushärtung durch periodisches Messen der **Shore D-Härte** nach DIN 53505 verfolgt.

Zur Prüfung der mechanischen Eigenschaften wurde die Vergussmasse als Film mit einer Schichtdicke von ca. 2 mm in eine plane PTFE-Form gegossen, der Film während 7 Tagen im Normklima ausgehärtet und nach DIN EN 53504 auf **Zugfestigkeit, Bruchdehnung** und **E-Modul** (bei 0.5 - 3.0 % Dehnung, Zuggeschwindigkeit: 10 mm/min) geprüft.

Die **Blasenbildung** wurde qualitativ beurteilt anhand der Menge Blasen, die bei der Aushärtung eines Films mit einer Schichtdicke von 2 mm im Normklima auftraten.

Die Ergebnisse dieser Prüfungen sind in Tabelle 2 aufgeführt.

**Tabelle 2: Eigenschaften der zweikomponentigen Vergussmassen.**

| Beispiel | **6** | **7** | **8** | **9** | **10** **(Vgl)** | **11** **(Vgl)** |
|---|---|---|---|---|---|---|
| Klebefreiheit (min.)^{a} | 21 | 19 | 18 | 42 | 58 | 48 |
| Shore D nach 1 Tag | 81 | 78 | 83 | 78 | 60 | 60 |
| Shore D nach 3 Tagen | 93 | 93 | 89 | 87 | 73 | 75 |
| Shore D nach 7 Tagen | 93 | 94 | 93 | 93 | 84 | 82 |
| Shore D getempert^{b} | 93 | 96 | 93 | 94 | 86 | 85 |
| Zugfestigkeit (MPa) | 23.4 | 26.7 | 25.5 | 13.7 | 11.0 | 8.1 |
| Bruchdehnung (%) | 6 | 3 | 5 | 23 | 75 | 60 |
| E-Modul (MPa) | 580 | 780 | 615 | 290 | 85 | 100 |
| Blasenbildung | keine | keine | keine | keine | keine | viele |

| | | | | | | |
|---|---|---|---|---|---|---|
| ^{a} Zeit bis zur Klebefreiheit (tack-free time). ^{b} 4 h bei 105°C der während 7 Tagen im Normklima ausgehärteten Prüfkörper. | | | | | | |

### Beispiele 12 bis 14: Semistrukturelle 2K-Klebstoffe

Für jedes Beispiel wurden die jeweiligen Bestandteile der Komponente ***K2*** gemäss Tabelle 3 in den angegebenen Gewichtsteilen ohne vorherige Trocknung in einen Polypropylenbecher mit Schraubverschluss eingewogen und mittels eines Zentrifugalmischers (SpeedMixer™ DAC 150, FlackTek Inc.; 2 min. bei 3000 U/min) zu einer homogenen Creme gemischt. Dazu wurden die in Tabelle 3 angegebenen Gewichtsteile an PMDI als Komponente ***K1*** zugegeben und eingemischt (30 sec. bei 3000 U/min). Das Verhältnis zwischen den Isocyanatgruppen der Komponente ***K1*** und der Summe der Reaktivgruppen (Hydroxyl- und Aldiminogruppen) der Komponente ***K2*** beträgt stets 1.1.

**Tabelle 3: Zusammensetzung der semistrukturellen 2K-Klebstoffe.**

| Beispiel | **12** | **13** | **14** |
|---|---|---|---|
| **Komponente *K1*:** | | | |
| PMDI^{a} | 24.5 | 28.0 | 19.0 |

| **Komponente *K2*:** | | | |
|---|---|---|---|
| Ricinusöl^{b} | 22.4 | 22.4 | 22.4 |
| PPG 1000^{c} | 22.4 | 22.4 | 22.4 |
| Triol^{d} | 2.25 | 2.25 | 2.25 |
| Aldimin | ***A-1,*** 5.0 | ***A-2,*** 5.0 | ***A-3*,** 5.0 |
| Säurekatalysator^{e} | 0.25 | 0.25 | 0.25 |
| Kreide^{f} | 50 | 50 | 50 |

| | | | |
|---|---|---|---|
| ^{a} Desmodur^{®} VKS 20 F, Bayer; NCO-Gehalt = 30.0 Gew.-%. ^{b} OH-Zahl = 165 mg KOH/g. ^{c} Desmophen^{®} 1112 BD, Bayer; OH-Zahl = 112 mg KOH/g. ^{d} Desmophen^{®} 4011 T, Bayer; OH-Zahl = 550 mg KOH/g. ^{e} Salicylsäure (5 Gew.-% in Dioctyladipat). ^{f} Omyacarb^{®} 5-GU, Omya. | | | |

Die so erhaltenen Klebstoffe wurden auf Aushärtegeschwindigkeit, mechanische Eigenschaften und Blasenbildung geprüft wie bei Beispiel 6 beschrieben. Die Ergebnisse der Prüfungen sind in Tabelle 4 aufgeführt.

**Tabelle 4: Eigenschaften der semistrukturellen 2K-Klebstoffe.**

| Beispiel | **12** | **13** | **14** |
|---|---|---|---|
| Klebefreiheit (min.)^{a} | 28 | 38 | 42 |
| Shore D nach 1 Tag | 65 | 73 | 62 |
| Shore D nach 3 Tagen | 77 | 81 | 74 |
| Shore D nach 7 Tagen | 82 | 86 | 82 |
| Shore D getempert^{b} | 84 | 87 | 86 |
| Zugfestigkeit (MPa) | 10.9 | 13.1 | 10.2 |
| Bruchdehnung (%) | 42 | 25 | 43 |
| E-Modul (MPa) | 109 | 186 | 100 |
| Blasenbildung | keine | keine | keine |

| | | | |
|---|---|---|---|
| ^{a} Zeit bis zur Klebefreiheit (tack-free time) in Minuten. ^{b} 4 h bei 105°C der während 7 Tagen im Normklima ausgehärteten Prüfkörper. | | | |

### Beispiele 15 und 16:

### Elastische 2K-Beschichtungen (z.B. für Bodenbelag)

Für jedes Beispiel wurden die jeweiligen Bestandteile der Komponente ***K1*** gemäss Tabelle 5 in den angegebenen Gewichtsteilen ohne vorherige Trocknung in eine Polypropylenkartusche eingewogen und mittels eines Zentrifugalmischers (SpeedMixer™ DAC 150, FlackTek Inc.; 30 sec. bei 2500 U/min) gemischt. Dazu wurden die angegebenen Gewichtsteile des Aldimins gemäss Tabelle 4 als Komponente ***K2*** zugegeben und eingemischt (30 sec. bei 2500 U/min). Das Verhältnis zwischen den Isocyanatgruppen der Komponente ***K1*** und der Summe der Reaktivgruppen (Hydroxyl- und Aldiminogruppen) der Komponente ***K2*** beträgt stets 1.1.

Das Polyurethanpolymer *1* wurde wie folgt hergestellt:
1060 g Polyoxypropylen-Diol (Desmophen^{®} 1111 BD, Bayer; OH-Zahl 111.4 mg KOH/g), 650 g Polyoxypropylen-Diol (Desmophen^{®} 2061 BD, Bayer; OH-Zahl 56.1 mg KOH/g), 770 g Isophorondiisocyanat (Vestanat^{®} IPDI, Degussa) und 0.25 g Dibutylzinndilaurat wurden bei 80 °C zu einem NCOterminierten Polyurethanpolymeren mit einem Gehalt an freien IsocyanatGruppen von 6.8 Gewichts-% umgesetzt.

**Tabelle 5: Zusammensetzung der zweikomponentigen Beschichtungen.**

| Beispiel | **15** | **16** |
|---|---|---|
| **Komponente *K1*:** | | |
| Polyurethanpolymer 1 | 64.0 | 64.0 |
| IPDI-Trimerisat^{a} | 32.0 | 32.0 |
| Säurekatalysator^{b} | 1.0 | 1.5 |
| Aminkatalysator^{c} | 0.5 | 0.5 |
| Zinnkatalysator^{d} | 1.0 | 1.0 |
| Entschäumer^{e} | 1.5 | 1.5 |

| **Komponente *K2*:** | | |
|---|---|---|
| Aldimin | ***A-2,*** 13.6 | ***A-3*,** 15.3 |

| | | |
|---|---|---|
| ^{a} 45 Gew.-% IPDI-Trimerisat (Vestanat^{®} T 1890/100, Degussa; NCO-Gehalt = 17.3 Gew.-%) in Xylol. ^{b} 5 Gew.-% Salicylsäure in Dioctyl-adipat. ^{c} 2,2'-Dimorpholinodiethylether (DABCO^{®} DMDEE Catalyst, Air Products). ^{d} 10 Gew.-% Dibutylzinn-dilaurat in Diisodecylphthalat. ^{e} BYK-088 (BYK-Chemie/ALTANA). | | |

Die so erhaltenen Beschichtungen wurden auf Zeit bis zur Klebefreiheit, auf mechanische Eigenschaften nach der Aushärtung sowie auf Blasenbildung wie bei Beispiel 6 beschrieben geprüft. Zusätzlich wurde die **Geruchsbildung** durch durch Riechen mit der Nase in einem Abstand von 10 cm an einem ausgehärteten Film qualitativ beurteilt.

Die Ergebnisse dieser Prüfungen sind in Tabelle 6 aufgeführt.

**Tabelle 6: Eigenschaften der zweikomponentigen Beschichtungen.**

| Beispiel | **15** | **16** |
|---|---|---|
| Klebefreiheit (min.)^{a} | 110 | 185 |
| Shore D nach 28 Tagen | 65 | 50 |
| Zugfestigkeit (MPa) | 9.3 | 8.3 |
| Bruchdehnung (%) | 190 | 310 |
| E-Modul (MPa)^{b} | 115 | 46 |
| Blasenbildung | keine | keine |
| Geruchsbildung | keine | keine |

| | | |
|---|---|---|
| ^{a} Zeit bis zur Klebefreiheit (tack-free time) in Minuten. ^{b} bei 0.5-5.0% Dehnung. | | |

## Patentansprüche

1. Aldimin der Formel (I) 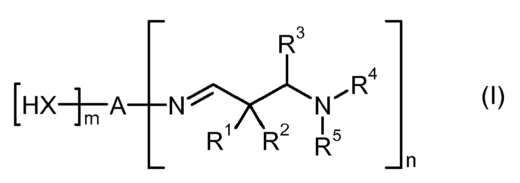 wobei
A entweder
für den Rest eines Amins nach Entfernung von n primären aliphatischen Aminogruppen und m HX-Gruppen steht,
oder
zusammen mit R⁷ für einen (n+2)-wertigen Kohlenwasserstoffrest mit 3 bis 20 C-Atomen, welcher gegebenenfalls mindestens ein Heteroatom, insbesondere in der Form von Ether-Sauerstoff oder tertiärem Amin-Stickstoff, enthält, steht;
n für 1 oder 2 oder 3 oder 4 steht;
m für 0 oder 1 oder 2 oder 3 oder 4 steht;
R¹ und R² entweder
unabhängig voneinander jeweils für einen einwertigen Kohlenwasserstoffrest mit 1 bis 12 C-Atomen stehen
oder
zusammen für einen zweiwertigen Kohlenwasserstoffrest mit 4 bis 12 C-Atomen, der Teil eines, gegebenenfalls substituierten, carbocyclischen Rings mit 5 bis 8, bevorzugt 6, C-Atomen ist, stehen;
R³ für ein Wasserstoffatom oder eine Alkylgruppe oder eine Arylalkylgruppe oder eine Alkoxycarbonylgruppe steht;
R⁴ und R⁵ entweder
unabhängig voneinander jeweils für eine Methylgruppe oder einen einwertigen aliphatischen, cycloaliphatischen oder arylaliphatischen Rest mit 2 bis 12 C-Atomen stehen, welcher gegebenenfalls Hydroxylgruppen aufweist, und welcher gegebenenfalls Heteroatome in der Form von Ether-Sauerstoff oder tertiärem Amin-Stickstoff enthält, mit der Massgabe, dass R⁴ mindestens eine Hydroxylgruppe aufweist,
oder
zusammen für einen mindestens eine Hydroxylgruppe aufweisenden zweiwertigen aliphatischen Rest mit 4 bis 12 C-Atomen, der Teil eines, gegebenenfalls substituierten, heterocyclischen Rings mit 5 bis 8, bevorzugt 6, Ringatomen ist, wobei dieser Ring gegebenenfalls weitere Heteroatome in der Form von Ether-Sauerstoff oder tertiärem Amin-Stickstoff enthält, stehen;
X für O oder S oder N-R⁶ oder N-R⁷ steht,
wobei R⁶
entweder für einen einwertigen Kohlenwasserstoffrest mit 1 bis 20 C-Atomen, welcher gegebenenfalls mindestens eine Carbonsäureester-, Nitril-, Nitro-, Phosphonsäureester-, Sulfon- oder Sulfonsäureester-Gruppe aufweist, steht,
oder
für einen Substituenten der Formel (II) steht, 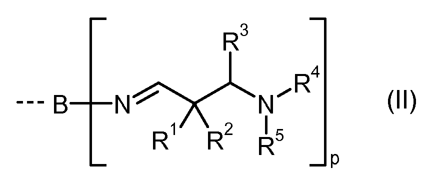 wobei
p für 0 oder für eine ganze Zahl von 1 bis 10'000 steht, und B für einen (p+1)-wertigen Kohlenwasserstoffrest, welcher gegebenenfalls Ether-Sauerstoff, tertiären Amin-Stickstoff, Hydroxylgruppen, sekundäre Aminogruppen oder Mercaptogruppen enthält, steht; und
R⁷ zusammen mit A für einen (n+2)-wertigen Kohlenwasserstoffrest mit 3 bis 20 C-Atomen, welcher gegebenenfalls mindestens ein Heteroatom, insbesondere in Form von Ether-Sauerstoff oder tertiärem Amin-Stickstoff, enthält, steht.

2. Aldimin gemäss Anspruch 1, **dadurch gekennzeichnet, dass** R¹ und R² jeweils für eine Methylgruppe stehen.

3. Aldimin gemäss Anspruch 1 oder 2, **dadurch gekennzeichnet, dass** R³ für ein Wasserstoffatom steht.

4. Aldimin gemäss einem der vorhergehenden Ansprüche, **dadurch gekennzeichnet, dass** R⁴ und R⁵ jeweils für eine 2-Hydroxyethylgruppe oder je für eine 2-Hydroxypropylgruppe stehen.

5. Aldimin gemäss einem der vorhergehenden Ansprüche, **dadurch gekennzeichnet, dass** A für den Rest eines Amins **B1,** welches ausgewählt ist aus der Gruppe bestehend aus N-Methyl-1,2-ethandiamin, N-Ethyl-1,2-ethandiamin, N-Cyclohexyl-1,2-ethandiamin, N-Methyl-1,3-propandiamin, N-Ethyl-1,3-propandiamin, N-Butyl-1,3-propandiamin, N-Cyclohexyl-1,3-propandiamin, 4-Aminomethyl-piperidin, 3-(4-Aminobutyl)-piperidin, Diethylentriamin (DETA), Dipropylentriamin (DPTA), Bis-hexamethylentriamin (BHMT), Fettdiamine wie N-Cocoalkyl-1,3-propandiamin, N-Oleyl-1,3-propandiamin, N-Soyaalkyl-1,3-propandiamin und N-Talgalkyl-1,3-propandiamin, 5-Amino-1-pentanol, 6-Amino-1-hexanol, 4-(2-Aminoethyl)-2-hydroxyethylbenzol, 3-Aminomethyl-3,5,5-trimethyl-cyclohexanol, 2-(2-Aminoethoxy)-ethanol, Triethylenglykol-monoamin, 3-(2-Hydroxyethoxy)-propylamin, 3-(2-(2-Hydroxyethoxy)-ethoxy)-propylamin und 3-(6-Hydroxyhexyloxy)-propylamin, steht.

6. Aldimin gemäss einem der vorhergehenden Ansprüche, **dadurch gekennzeichnet, dass** A für den Rest eines Amins **B2,** welches ausgewählt ist aus der Gruppe bestehend aus 1,6-Hexamethylendiamin, 1,5-Diamino-2-methylpentan (MPMD), 1,3-Pentandiamin (DAMP), 1-Amino-3-aminomethyl-3,5,5-trimethylcyclohexan (= Isophorondiamin oder IPDA), 2,2,4-und 2,4,4-Trimethylhexamethylendiamin (TMD), 1,3-Xylylendiamin, 1,3-Bis-(aminomethyl)cyclohexan, Bis-(4-aminocyclohexyl)-methan, Bis-(4-amino-3-methylcyclohexyl)-methan, 3(4),8(9)-Bis-(aminomethyl)-tricyclo-[5.2.1.0^{2,6}]decan, 1,2-, 1,3- und 1,4-Diaminocyclohexan, 1,4-Diamino-2,2,6-trimethylcyclohexan, 3,6-Dioxaoctan-1,8-diamin, 4,7-Dioxadecan-1,10-diamin, 4-Aminomethyl-1,8-octandiamin und Polyoxyalkylen-Polyamine mit zwei oder drei Aminogruppen, steht.

7. Aldimin gemäss einem der vorhergehenden Ansprüche, **dadurch gekennzeichnet, dass** das Aldimin die Formel (I') aufweist, 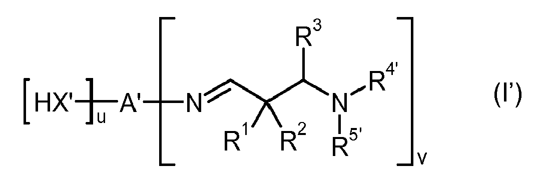 wobei
A' entweder
für den Rest eines Amins nach Entfernung von v primären aliphatischen Aminogruppen und u HX'-Gruppen steht,
oder
zusammen mit R^{7'} für einen (v+2)-wertigen Kohlenwasserstoffrest mit 3 bis 20 C-Atomen, welcher gegebenenfalls mindestens ein Heteroatom, insbesondere in der Form von Ether-Sauerstoff oder tertiärem Amin-Stickstoff, enthält, steht;
u für 1 oder 2 oder 3 oder 4 steht, und
v für 0 oder 1 oder 2 oder 3 oder 4 steht,
mit der Massgabe, dass u+v für 2 oder 3 oder 4 oder 5 steht;
R^{4'} und R^{5'} entweder
unabhängig voneinander jeweils für eine Methylgruppe oder einen einwertigen aliphatischen, cycloaliphatischen oder arylaliphatischen Rest mit 2 bis 12 C-Atomen stehen, welcher gegebenenfalls Hydroxylgruppen aufweist, und welcher gegebenenfalls Heteroatome in der Form von Ether-Sauerstoff oder tertiärem Amin-Stickstoff enthält, mit der Massgabe, dass R^{4'} mindestens eine Hydroxylgruppe aufweist und dass R^{4'} und R^{5'} zusammen mindestens zwei Hydroxylgruppen aufweisen,
oder
zusammen für einen mindestens zwei Hydroxylgruppen aufweisenden zweiwertigen aliphatischen Rest mit 4 bis 12 C-Atomen, der Teil eines, gegebenenfalls substituierten, heterocyclischen Rings mit 5 bis 8, bevorzugt 6, Ringatomen ist, wobei dieser Ring gegebenenfalls weitere Heteroatome in der Form von Ether-Sauerstoff oder tertiärem Amin-Stickstoff enthält, stehen;
X' für O oder S oder N-R^{6'} oder N-R^{7'} steht,
wobei R^{6'}
entweder für einen einwertigen Kohlenwasserstoffrest mit 1 bis 20 C-Atomen, welcher gegebenenfalls mindestens eine Carbonsäureester-, Nitril-, Nitro-, Phosphonsäureester-, Sulfon- oder Sulfonsäureester-Gruppe aufweist, steht,
oder
für einen Substituenten der Formel (II') steht 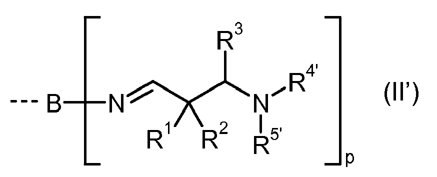
und
R^{7'} zusammen mit A' für einen (v+2)-wertigen Kohlenwasserstoffrest mit 3 bis 20 C-Atomen, welcher gegebenenfalls mindestens ein Heteroatom, insbesondere in Form von Ether-Sauerstoff oder tertiärem Amin-Stickstoff, enthält, steht.

8. Aldimin gemäss Anspruch 7, **dadurch gekennzeichnet, dass** (u+v) für 2 oder 3 steht.

9. Aldimin gemäss Anspruch 7 oder 8, **dadurch gekennzeichnet, dass** entweder
R^{4'} zwei Hydroxylgruppen und R^{5'} keine Hydroxylgruppe aufweist;
oder
R^{4'} eine Hydroxylgruppe und R^{5'} eine Hydroxylgruppe aufweist.

10. Aldimin gemäss Anspruch 7 oder 8 oder 9, **dadurch gekennzeichnet, dass** das Aldimin die Formel (I a) aufweist, 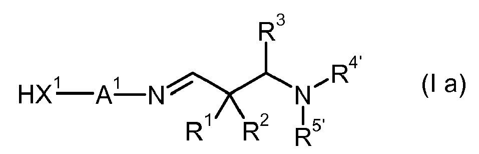 wobei
A¹ keinen aktiven Wasserstoff und keine primären Aminogruppen aufweist und
entweder
für einen zweiwertigen Kohlenwasserstoffrest mit 2 bis 20 C-Atomen, welcher gegebenenfalls mindestens ein Heteroatom, insbesondere in der Form von Ether-Sauerstoff oder tertiärem Amin-Stickstoff, enthält, steht,
oder
zusammen mit R⁹ für einen dreiwertigen Kohlenwasserstoffrest mit 3 bis 20 C-Atomen, welcher gegebenenfalls mindestens ein Heteroatom, insbesondere in der Form von Ether-Sauerstoff oder tertiärem Amin-Stickstoff, enthält, steht;
X¹ für O oder S oder N-R⁸ oder N-R⁹ steht,
wobei R⁸
entweder für einen einwertigen Kohlenwasserstoffrest mit 1 bis 20 C-Atomen, welcher gegebenenfalls mindestens eine Carbonsäureester-, Nitril-, Nitro-, Phosphonsäureester-, Sulfon- oder Sulfonsäureester-Gruppe aufweist, steht,
oder
für einen Substituenten der Formel (II a) steht, 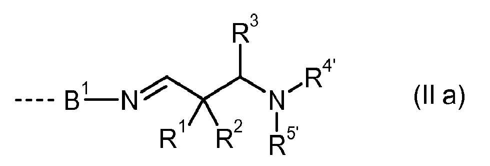 wobei B¹ für einen zweiwertigen, gegebenenfalls Ether-Sauerstoff oder tertiären Amin-Stickstoff aufweisenden Kohlenwasserstoffrest mit 2 bis 12 C-Atomen steht; und
R⁹ zusammen mit A¹ für einen dreiwertigen Kohlenwasserstoffrest mit 3 bis 20 C-Atomen, welcher gegebenenfalls mindestens ein Heteroatom, insbesondere in der Form von Ether-Sauerstoff oder tertiärem Amin-Stickstoff, enthält, steht.

11. Aldimin gemäss Anspruch 7 oder 8 oder 9, **dadurch gekennzeichnet, dass** das Aldimin die Formel (I b) aufweist, 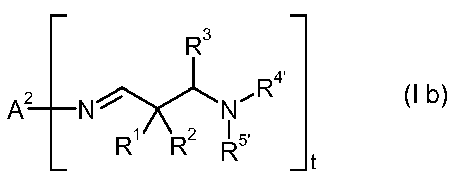 wobei t für 2 oder 3 steht; und
A² für den Rest eines Polyamins mit t primären Aminogruppen nach Entfernung von t primären Aminogruppen steht und keinen aktiven Wasserstoff enthält.

12. Verfahren zur Herstellung eines Aldimins gemäss einem der Ansprüche 1 bis 11, **dadurch gekennzeichnet, dass** mindestens ein Amin **B** der Formel (III) mit mindestens einem sterisch gehinderten, aliphatischen, mindestens eine Hydroxylgruppe aufweisenden Aldehyd **ALD** der Formel (IV) umgesetzt wird, 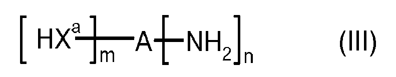 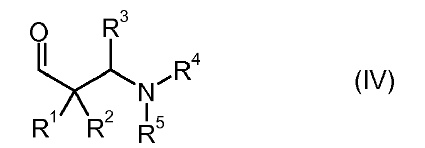 wobei X^{a} für O oder S oder N-R^{6a} oder N-R⁷steht, wobei R^{6a} entweder für einen einwertigen Kohlenwasserstoffrest mit 1 bis 20 C-Atomen, welcher gegebenenfalls mindestens eine Carbonsäureester-, Nitril-, Nitro-, Phosphonsäureester-, Sulfon- oder Sulfonsäureester-Gruppe aufweist, steht, oder für einen Substituenten der Formel (III') steht.

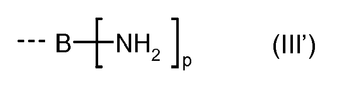

13. Verfahren gemäss Anspruch 12, **dadurch gekennzeichnet, dass** ein Aldehyd **Y1** der Formel (V), ein Aldehyd **Y2** der Formel (VI) und ein sekundäres, aliphatisches, mindestens eine Hydroxylgruppe aufweisendes Amin C der Formel (VII) unter Wasserabspaltung zum Aldehyd **ALD** der Formel (IV) umgesetzt werden.

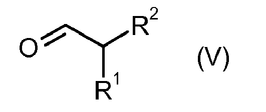

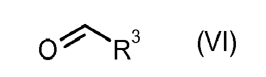

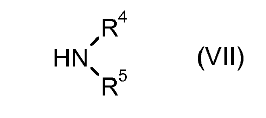

14. Verfahren gemäss Anspruch 13, **dadurch gekennzeichnet, dass** das mindestens eine Hydroxylgruppe aufweisende Amin **C** der Formel (VII) ein sekundäres aliphatisches Amin ist, welches mindestens zwei Hydroxylgruppe aufweist, und welches insbesondere ausgewählt ist aus der Gruppe bestehend aus Diethanolamin, Dipropanolamin, Diisopropanolamin, 3-(2-Hydroxyethylamino)-1-propanol und 3-(2-Hydroxypropyl-amino)-1-propanol, N-Methyl-2,3-dihydroxypropylamin, 3,4-Dihydroxypyrrolidin, 2,5-Bis-(hydroxymethyl)-pyrrolidin, 2,6-Bis-(hydroxymethyl)-piperidin, 3,4- oder 3,5-Dihydroxy-piperidin, 2-(2,3-Dihydroxypropyl)-pyrrolidin und 2-(2,3-Dihydroxypropyl)-piperidin sowie die Umsetzungsprodukte aus Ammoniak mit zwei Molekülen, welche je eine Epoxidgruppe, insbesondere eine Glycidylether-Gruppe, aufweisen.

15. Aldimin der Formel (IX) 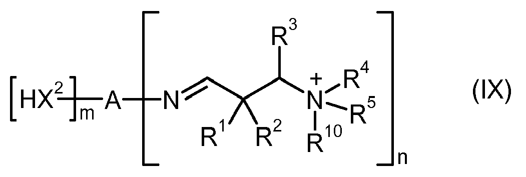 wobei R¹⁰ für ein Wasserstoffatom oder für einen Alkyl-, Cycloalkyl- oder Arylalkylrest mit 1 bis 20 C-Atomen steht;
und X² für O oder S oder N-R¹¹ oder N-R⁷ steht; wobei
R¹¹ entweder für einen einwertigen Kohlenwasserstoffrest mit 1 bis 20 C-Atomen, welcher gegebenenfalls mindestens eine Carbonsäureester-, Nitril-, Nitro-, Phosphonsäureester-, Sulfon- oder Sulfonsäureester-Gruppe aufweist, steht,
oder für einen Substituenten der Formel (IX') steht, 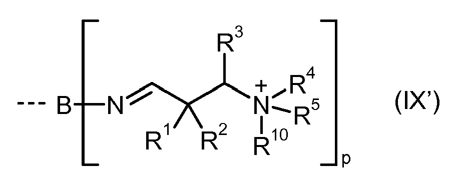 welches durch Protonierung oder Alkylierung von einem Aldimin der Formel (I) gemäss einem der Ansprüche 1 bis 11 erhältlich ist.

16. Verwendung eines Aldimin der Formel (I) gemäss einem der Ansprüche 1 bis 11 oder eines Aldimin der Formel (IX) gemäss Anspruch 15 als Bestandteil von Zusammensetzungen basierend auf Isocyanaten oder Epoxidharzen, insbesondere für Klebstoffe, Dichtstoffe, Vergussmassen, Beschichtungen, Bodenbeläge, Anstriche, Lacke, Primer und Schäume.

17. Härtbare Zusammensetzung enthaltend mindestens ein Polyisocyanat und mindestens ein Aldimin der Formel (I) gemäss einem der Ansprüche 1 bis 11 oder ein Aldimin der Formel (IX) gemäss Anspruch 15.

18. Härtbare Zusammensetzung enthaltend mindestens ein Polyisocyanat und mindestens ein Aldimin der Formel (I') gemäss einem der Ansprüche 7 bis 11.

19. Härtbare Zusammensetzung gemäss Anspruch 18, **dadurch gekennzeichnet, dass** sie eine einkomponentige Zusammensetzung ist, und dass sie mindestens ein Polyisocyanat, dessen Isocyanatgruppen als blockierte Isocyanatgruppen vorliegen, umfasst.

20. Härtbare Zusammensetzung gemäss Anspruch 18, **dadurch gekennzeichnet, dass** sie eine zweikomponentige Zusammensetzung bestehend aus einer Komponente **K1** und einer Komponente **K2** ist, wobei die Komponente **K1** mindestens ein Polyisocyanat **P** enthält.

21. Härtbare Zusammensetzung gemäss Anspruch 20, **dadurch gekennzeichnet, dass** das Polyisocyanat **P** ein Polyisocyanat **PI** in der Form eines monomeren Di- oder Triisocyanates oder eines Oligomeren eines monomeren Diisocyanates oder eines Derivates eines monomeren Diisocyanates, insbesondere von 1,6-Hexamethylendiisocyanat (HDl), 1-Isocyanato-3,3,5-trimethyl-5-isocyanatomethyl-cyclohexan (=Isophorondiisocyanat oder IPDI), 2,4- und 2,6-Toluylendiisocyanat und beliebige Gemische dieser Isomeren (TDI), oder 4,4'-, 2,4'- und 2,2'-Diphenylmethandiisocyanat und beliebige Gemische dieser Isomeren (MDI), ist.

22. Härtbare Zusammensetzung gemäss Anspruch 20, **dadurch gekennzeichnet, dass** das Polyisocyanat **P** ein Isocyanatgruppen aufweisendes Polyurethanpolymer **PUP** ist, welches insbesondere durch die Umsetzung von mindestens einem Polyol mit mindestens einem Polyisocyanat, insbesondere einem monomeren Diisocyanat, erhältlich ist.

23. Härtbare Zusammensetzung gemäss einem der Ansprüche 20 bis 22, **dadurch gekennzeichnet, dass** die Komponente **K2** Wasser enthält.

24. Ausgehärtete Zusammensetzung erhalten durch die Reaktion einer härtbaren Zusammensetzung gemäss einem der Ansprüche 17 bis 23 und Wasser, insbesondere in Form von Luftfeuchtigkeit.

25. Verfahren zum Verkleben von einem Substrat **S1** mit einem Substrat **S2,** welches die Schritte umfasst
i) Applikation einer härtbaren Zusammensetzung gemäss einem der Ansprüche 17 bis 23 auf ein Substrat **S1;**
ii) Kontaktieren der applizierten Zusammensetzung mit einem Substrat **S2** innerhalb der Offenzeit der Zusammensetzung
oder
i') Applikation einer Zusammensetzung gemäss einem der Ansprüche 17 bis 23 auf ein Substrat **S1** und auf ein Substrat **S2**
ii') Kontaktieren der applizierten Zusammensetzung miteinander innerhalb der Offenzeit der Zusammensetzung;
wobei das Substrat **S2** aus dem gleichen oder einem unterschiedlichen Material wie das Substrat **S1** besteht.

26. Verfahren zum Abdichten, welches den Schritt umfasst
i") Applikation einer härtbaren Zusammensetzung gemäss einem der Ansprüche 17 bis 23 zwischen ein Substrat **S1** und ein Substrat **S2,** so dass die Zusammensetzung mit dem Substrat **S1** und dem Substrat **S2** in Kontakt steht;
wobei das Substrat **S2** aus dem gleichen oder einem unterschiedlichen Material wie das Substrat **S1** besteht.

27. Verfahren zum Beschichten eines Substrates **S1,** welches den Schritt umfasst
i''') Applikation einer härtbaren Zusammensetzung gemäss einem der Ansprüche 17 bis 23 auf ein Substrat **S1** innerhalb der Offenzeit der Zusammensetzung.

28. Verfahren gemäss Anspruch 25 oder 26 oder 27, **dadurch gekennzeichnet, dass** das Substrat **S1** und/oder das Substrat **S2** ein anorganisches Substrat, wie Glas, Glaskeramik, Beton, Mörtel, Backstein, Ziegel, Gips oder Naturstein, wie Granit oder Marmor; ein Metall oder eine Legierung, wie Aluminium, Stahl, Buntmetall, verzinktes Metall; ein organisches Substrat, wie Holz, ein Kunststoff, wie PVC, Polycarbonat, PMMA, Polyethylen, Polypropylen, Polyester, Epoxidharz; ein beschichtetes Substrat, wie pulverbeschichtetes Metall oder Legierung; oder eine Farbe oder ein Lack, insbesondere ein Automobildecklack, ist.

29. Artikel, welcher nach einem Verfahren gemäss einem der Ansprüche 25 bis 28 verklebt, abgedichtet oder beschichtet wurde.

30. Artikel gemäss Anspruch 29, **dadurch gekennzeichnet, dass** der Artikel ein Bauwerk, insbesondere ein Bauwerk des Hoch- oder Tiefbaus, oder ein industrielles Gut oder ein Konsumgut, insbesondere ein Fenster, eine Haushaltmaschine, oder ein Transportmittel, insbesondere ein Fahrzeug zu Wasser oder zu Lande, bevorzugt ein Automobil, ein Bus, ein Lastkraftwagen, ein Zug oder ein Schiff, oder ein Anbauteil eines Transportmittels, oder ein Artikel der Möbel-, Textil- oder Verpackungsindustrie ist.
